# EUROPEAN PATENT SPECIFICATION

(11) **EP 2 083 016 B1**
(45) Date of publication and mention of the grant of the patent: **15.06.2011**
(21) Application number: 09001558.7
(22) Date of filing: 26.11.2001
(51) Int. Cl.: C07K 14/315, C12N 15/62, C07K 19/00, A61K 39/09, C07K 16/12

(54) **Bacterial superantigen vaccines**
Bakterielle Superantigenimpfstoffe
Vaccins super-antigènes bactériens

(43) Date of publication of application: 29.07.2009
(62) Divisional of application: 01996119.2
(73) Proprietor: U.S. Medical Research Institute of Infectious Diseases, Frederick, MD 21702 (US)
(72) Inventor: Ulrich, Robert G., Frederick, MD 21702 (US)
(74) Representative: Fairbairn, Angus Chisholm

(56) References cited:
- WO-A-00/09154
- WO-A-98/24911
- KLINE J B ET AL: "ANALYSIS OF THE SUPERANTIGENIC ACTIVITY OF MUTANT AND ALLELIC FORMSOF STREPTOCOCCAL PYROGENIC EXOTOXIN A" INFECTION AND IMMUNITY, AMERICAN SOCIETY FOR MICROBIOLOGY. WASHINGTON, vol. 64, no. 3, 1 March 1996 (1996-03-01), pages 861-869, XP002066999 ISSN: 0019-9567
- ROGGIANI MANUELA ET AL: "Toxoids of streptococcal pyrogenic exotoxin A are protective in rabbit models of streptococcal toxic shock syndrome" INFECTION AND IMMUNITY, vol. 68, no. 9, September 2000 (2000-09), pages 5011-5017, XP002531911 ISSN: 0019-9567
- TODD F. KAGAWA ET AL.: "Crystal structure of the zymogen form of the group A Streptococcus virulence factor SpeB: An integrin-binding cysteine protease" PNAS, vol. 97, no. 5, 29 February 2000 (2000-02-29), pages 2235-2240, XP002149908
- JOSEPH Y. TAI ET AL.: "Primary structure of Streptococcal proteinase. III. Isolation of cyanogen bromide peptides: Complete covalent structure of the polypeptide chain" THE JOURNAL OF BIOLOGICAL CHEMISTRY, vol. 251, no. 7, 10 April 1976 (1976-04-10), pages 1955-1959, XP001038607

## Description

### INTRODUCTION

Staphylococcal enterotoxins (SEs) A through E are the most commom cause of food poisoning [Bergdoll, M.S.(1983) In Easom CSF, Aslam C., eds. Staphylococci and staphylococcal infections. London: Academic Press, pp 559-598] and are associated with several serious diseases [Schlievert, P.M. (1993) J. Infect. Dis. 167: 997-1002; Ulrich et al. (1995) Trends Microbiol. 3: 463-468], such as bacterial arthritis [Schwab et al. (1993) J. Immunol. 150; 4151-4159; Goldenberg et al. (1985) N. Engl. J. Med. 312: 764-771], other autoimmune disorders [Psnett, D. N. (1993) Semin. Immunol. 5: 65-72], and toxic shock syndrome [Schlieverst, P.M. (1986) Lancet 1: 1149-1150; Bohach et al. (1990) Crit. Rev. Microbiol. 17: 251-272]. The nonenterotoxic staphylococcal superantigen toxic shock syndrome toxin-1 (TSST-1) was first identified as a causative agent of menstrual-associated toxic shock syndrome [Schlievert et al. (1981) J. Infect. Dis. 143: 509-516]. Superantigen-producing *Staphylococcus* aureus strains are also linked to Kawasaki syndrome, an inflammatory disease of children [Leung et al. (1993) Lancet 342: 1385-1388].

The staphylococcal enterotoxins A-E, toxic shock syndrome toxin-1 .(TSST-1), and streptococcal pyrogenic exotoxins A-C are soluble 23-29-kD proteins commonly referred to as bacterial superantigens (SAgs). Bacterial superantigens are ligands for both major histocompatibility complex (MHC) class II molecules, expressed on antigen-presenting cells, and the variable portion of the T cell antigen receptor β chain (TCR Vβ) [Choi et al. (1989) Proc. Natl. Acad. Sci. USA 86:8941-8945; Fraser, J.D. (1989) Nature 339:221-223; Marrack et al. (1990) Science 248: 705-711; Herman et al. (1991) Annu. Rev. Immunol. 9: 745-772; Mollick et al. (1989) Science 244:817-820].

Each bacterial superantigen has a distinct affinity to a set of TCR Vβ, and coligation of the MHC class II molecule polyclonally stimulates T cells [White et al. (1989) Cell 56: 27-35; Kappler et al. (1989) Science 244: 811-813; Takimoto et al. (1990) Eur J. Immunol. 140: 617-621]. Pathologically elevated levels of cytokines that are produced by activated T cells are the probable cause of toxic shock symptoms [Calson et al. (1985) Cell. Immunol. 96: 175-183; Stiles et al. (1993) Infect. Immun. 61: 5333-5338]. In addition, susceptibility to lethal gram-negative endotoxin shock is enhanced by several bacterial superantigens [Stiles, *et al.*, *supra*]. Although antibodies reactive with superantigens are present at low levels in human sera [Takei et al. (1993) J. Clin. Invest. 91: 602-607], boosting antibody titers by specific immunization may be efficacious for patients at risk for toxic shock syndrome and the other disorders of common etiology.

A vaccine approach to controlling bacterial superantigen-associated diseases presents a unique set of challenges. Acute exposure to bacterial superantigens produces T cell anergy, a state of specific non-responsiveness [Kawabe et al. (1991) Nature 349: 245-248], yet T cell help is presumably a requirement for mounting an antibody response.

Presently, the only superantigen vaccines available are chemically inactivated toxoids from different bacteria which have several disadvantages. The chemical inactivation process can be variable for each production lot making the product difficult to characterize. The chemical used for inactivation, (e.g. formaldehyde), is often toxic and does not negate the possibility of reversion of the inactivated superantigen to an active form. In addition, the yields of wild-type toxin from bacterial strains used for making toxoids are often low.

WO-A-00/9154 discloses various superantigen toxins, and specifically a streptococcal pyrogenic exotoxin-A (SpeA) in which position 42 has been altered to arginine whereby binding to MHC class II receptor is altered.

The present invention relates to a vaccine which overcomes the disadvantages of the chemically inactivated toxoids described above. The superantigen vaccine(s) of the present invention is/are designed to protect individuals against the pathologies resulting from exposure to one or several related staphylococcal and streptococcal toxins. The superantigen vaccine is comprised of a purified protein product that is genetically attenuated by DNA methodologies such that superantigen attributes are absent, however the superantigen is effectively recognized by the immune system and an appropriate antibody response is produced.

Specifically, the vaccine of the present invention is a product of site-directed mutagenesis of the DNA coding sequences of superantigen toxins resulting in a disruption of binding to both the MHC class II receptor and to the T-cell antigen receptor. A comprehensive study of the relationships of the superantigen structures of TSST-1, streptococcal pyrogenic exotoxin-A (SpeA), staphylococcal enterotoxin B (SEB), and staphylococcal enterotoxin A, to receptor binding were undertaken to provide insight into the design of the vaccine. From these studies, critical amino acid residues of the toxin responsible for binding the superantigen to the human MHC receptors were defined. Site-directed mutagenesis of the gene encoding the toxin and expression of the new protein product resulted in a superantigen toxin with disrupted binding to the MHC receptors.

Therefore, the present invention provides an isolated and purified superantigen toxin DNA fragment as defined in claims 1-2. Such a DNA fragment is useful in the production of a vaccine against superantigen toxin infections.

The present invention further provides an isolated and purified superantigen toxin as defined in claims 5-6. Such a sequence is useful for the production of a superantigen toxin vaccine.

The present invention further provides a recombinant DNA construct as defined in claims 3-4, comprising a vector and the DNA fragment described above.

The present invention may be useful to provide host cells transformed with the above-described recombinant DNA constructs. Host cells include cells of other prokaryotic species or eukaryotic plant or animal species, including yeasts, fungi, plant culture, mammalian and nonmammalian cell lines, insect cells and transgenic plants or animals.

The present invention may be useful to provide a method for producing altered superantigen toxin with disrupted MHC class II and T-cell antigen receptor binding which comprises culturing a host cell under conditions such that a recombinant vector comprising a vector and the DNA fragment described above is expressed and altered superantigen toxin is thereby produced, and isolating superantigen toxin for use as a vaccine against superantigen toxin-associated bacterial infections and as a diagnostic reagent.

The invention further provides an altered superantigen toxin as defined in claim 9, useful as a vaccine and as a diagnostic agent.

The invention may be useful to provide a purified altered superantigen toxin for the therapeutic stimulation of, or other *in vivo* manipulation of, selective T cell subsets, or *ex vivo* manipulation of T cells for *in vivo* therapeutic purposes in mammals. Diseases, such as autoimmunity, wherein T-cell responses of limited diversity (oligoclonal) are evident. Altered superantigens may be used to therapeutically inactivate (induce anergy in) T cells in diseases wherein oligoclonal T-cell responses are evident such as autoimmune diseases, for example. For diseases in which specific T-cell subsets are not active or are anergetic, altered superantigens may be used to therapeutically stimulate these T cells. Such disease include, but are not limited to, infectious diseases and cancers wherein specific subsets of cytotoxic or helper T cells are inactivated or are otherwise unable to respond normally to the antigenic stimulation of the disease moiety.

The present invention further provides antisera as defined in claims 10-12.

The present invention further provides an antibody to the above-described altered superantigen toxin, as defined in claims 13-15, useful as a therapeutic agent and as a diagnostic agent.

The invention further provides a superantigen toxin vaccine as defined in claim 8, comprising an altered superantigen toxin effective for the production of antigenic and immunogenic response resulting in the protection of an animal against superantigen toxin infection.

The invention further provides a multivalent vaccine as defined in claim 7. A multivalent superantigen toxin vaccine comprising altered toxins from a variety of streptococcal and staphylococcal toxins may be effective for the production of antigenic and immunogenic response resulting in the protection of an animal against infection with bacterial superantigen toxin-expressing strains and against other direct or indirect exposures to bacterial superantigen toxins such as might occur by ingestion, inhalation, injection, transdermal or other means.

The present invention may be useful to provide a method for the diagnosis of superantigen toxin-associated bacterial infection comprising the steps of:
(i) contacting a sample from an individual suspected of having a superantigen toxin-associated bacterial infection with antibodies which recognize superantigen toxin using antibodies generated from the altered superantigen toxin; and
(ii) detecting the presence or absence of a superantigen-associated bacterial infection by detecting the presence or absence of a complex formed between superantigen toxin in said sample and antibodies specific therefor.

The present invention may be useful to provide a method for the diagnosis of superantigen bacterial infection comprising the steps of:
(i) contacting a sample from an individual suspected of having the disease with lymphocytes which recognize superantigen toxin produced by said superantigen bacteria or lymphocytes which recognize altered superantigen toxin; and
(ii) detecting the presence or absence of responses of lymphocytes resulting from recognition of superantigen toxin. Responses can be, for example, measured cytokine release, increase of activation markers, mitotic activity, or cell lysis. The lymphocytes responding to the altered superantigen toxins recognize them as recall antigens not as superantigens, therefore the response is an indicator of prior exposure to the specific superantigen. The absence of a response may indicate no prior exposure, a defective immune response or in some cases a manifestation of T-cell anergy. Anergy is defined here as antigen-specific or a generalized non-responsiveness of subsets of T cells.

The present invention may be useful to provide a diagnostic kit comprising an antibody against an altered superantigen toxin and ancillary reagents suitable for use in detecting the presence of superantigen toxin in animal tissue or serum.

The present invention may be useful to provide a detection method for detecting superantigen toxins or antibodies to superantigen toxin in samples, said method comprising employing a biosensor approach. Such methods are known in the art and are described for example in Karlsson et al. (1991) J. Immunol. Methods 145, 229-240 and Jonsson et al. (1991) Biotechniques 11, 620-627.

The present invention may be useful to provide a therapeutic method for the treatment or amelioration of symptoms of superantigen-associated bacterial infection, said method comprising providing to an individual in need of such treatment an effective amount of sera from individuals immunized with one or more altered superantigen toxins from different bacteria in a pharmaceutically acceptable excipient.

The present invention may be useful to provide a therapeutic method for the treatment or amelioration of symptoms of superantigen toxin-associated bacterial infection, said method comprising providing to an individual in need of such treatment an effective amount of antibodies against altered superantigen toxins in a pharmaceutically acceptable excipient.

The present invention may be useful to provide a therapeutic method for the treatment or amelioration of symptoms of bacterial superantigen toxin infection, said method comprising providing to an individual in need of such treatment an effective amount of altered superantigen from a variety of streptococcal and staphylococcal bacteria in order to inhibit adhesion of superantigen bacterial toxin to MHC class II or T-cell receptors by competitive inhibition of these interactions.

The present invention may be useful to provide a therapeutic method for the treatment of diseases that may not be associated directly with superantigen toxins but which result in specific nonresponsiveness of T-cell subsets, said method comprising the administration of altered superantigen toxins, *in vivo* or *ex vivo*, such that T-cell subsets are expanded or stimulated. Diseases which cause anergy or nonresponsiveness of T-cells include, but are not limited to, infectious diseases.

The present invention may be useful to provide a therapeutic method for the treatment of diseases associated with expanded or over-stimulated T-cell subsets, such as autoimmunity for example, said method comprising administration of altered superantigen toxin, *in vivo* or *ex vivo*, such that anergy or inactivation of disease associated T-cells is produced. In this case, superantigen mutants can be designed with altered but not attenuated T-cell receptor binding, to cause anergy of only the select (i.e. 1-3) T-cell subsets that are pathologically activated.

### BRIEF DESCRIPTION OF THE DRAWINGS

These and other features, aspects, and advantages of the present invention will become better understood with reference to the following description and appended claims, and accompanying drawings where:
**Figure 1****.** Staphylococcal and streptococcal superantigen amino acid sequence homologies, compiled with Genetics Computers Group of Univ. of Wisconsin software.
**Figure 2****.** Comparison of mutant SEB and SEA biological activities.
   **A.** SEB mutant HLA-DR1-binding; **B.** SEA mutant HLA-DR1-binding; **C.** T-cell recognition of SEA and SEB mutants. Binding of bacterial superantigens to cell surface DR1 was measured by laser fluorescence-activated flow cytometry. A representative experiment of three performed is shown. The mutants SEA D197N, the homologous SEB D199N, and SEA L11Y had no effect on binding or T-cell recognition, and were used for controls. Human T-cell proliferation, assessed by [³H]thymidine incorporation, was measured in response to SEA (Y64A) or SEB (Y61A) mutants and controls that retained DR1-binding affinities. Each data point represents the mean of triplicate determinations; SEM 5%.

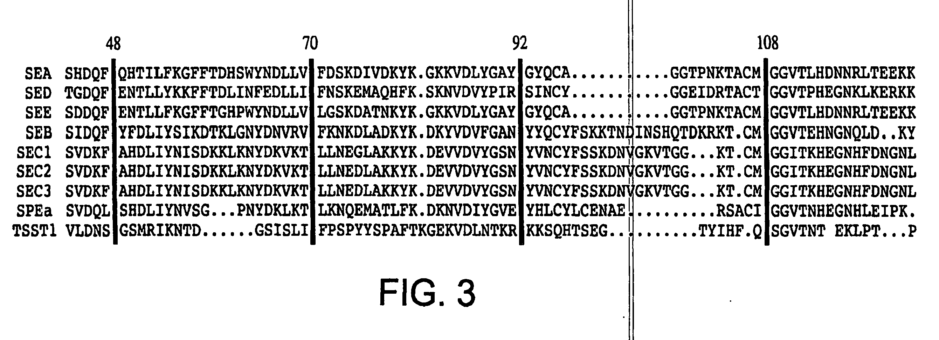

**Figure 3****.** Sequence and secondary structural alignment of bacterial superantigen toxins. Analyses were performed with the applications PILEUP and PROFILE from the Computer Genetics Group (Madison, WI) using sequence data obtained from a variety of sources. Amino acid residue numbering is based on the SEA sequence.

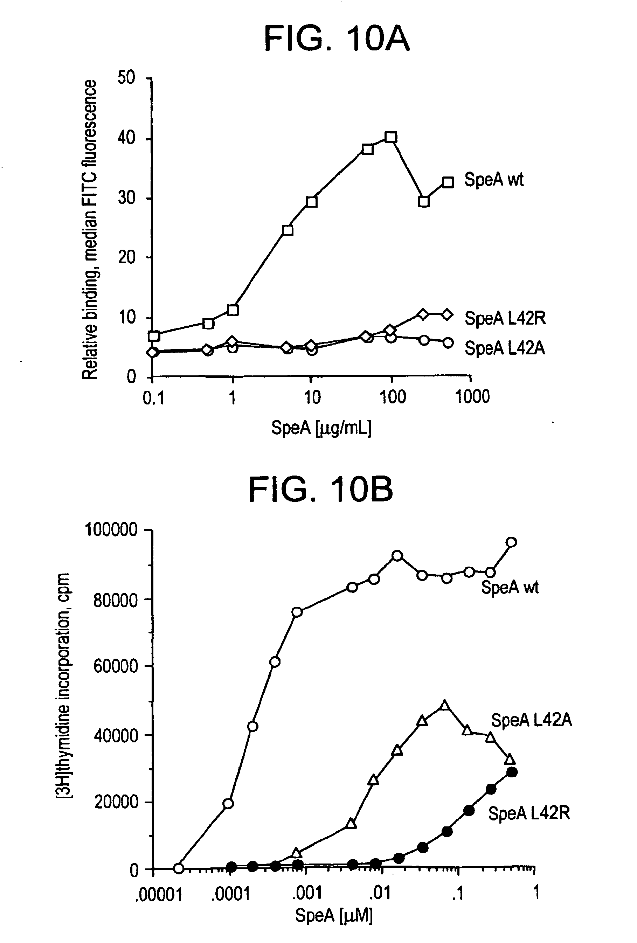

**Figure 10A, and 10B****.** Biological activities of SpeA mutants. A, Mutations of SpeA at amino acid position 42 (L42R) results in greatly diminished interactions with cell surface HLA-DR, measured by laser fluorescence-activated flow cytometry and FITC-labeled rabbit anti-SpeA antibody (affinity purified). B, Mutations of SpeA at amino acid position 42 (L42R or L42A) results in greatly diminished activation of human lymphocytes. Human T-cell proliferation, was assessed by [³H] thymidine incorporation, using a 12 h pulse with label and harvesting cells after 60 h of culture. Each data point represents the mean of triplicate determinations; SEM <5%.

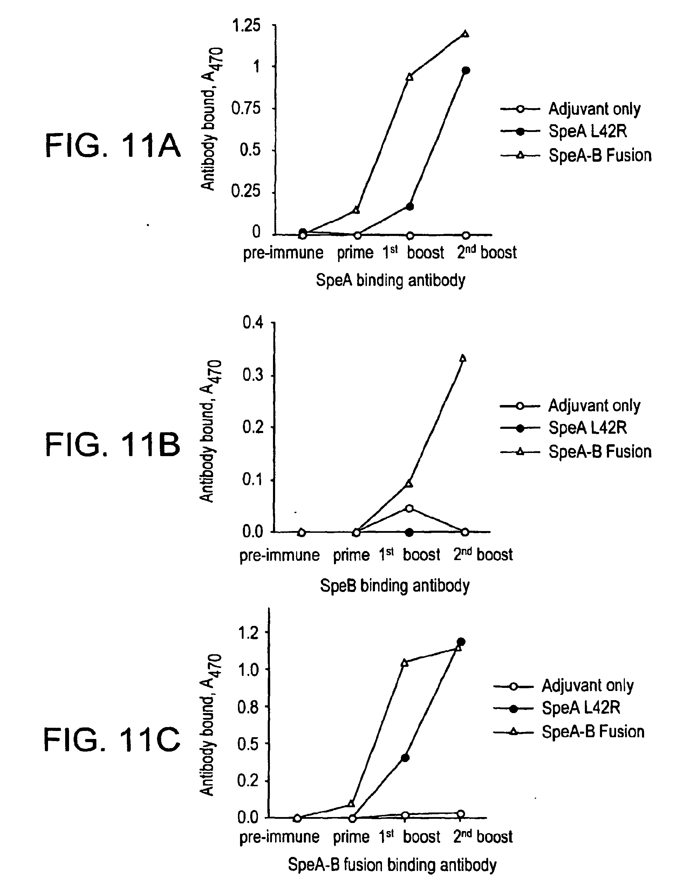

**Figure 11****.** Mouse antibody response to SpeA L42R and SpeA-B fusion constructs. BALB/c mice were vaccinated three times with 10 µg plus adjuvant (MPL™ + TDM+ CWS Emulsion, RIBI ImmunoCHem Research, Inc., Hamilton, MT) of each construct, allowing two weeks between injections. Sera from each experimental group (n=5) were pooled for measurement of specific antibodies. Data shown are antigen-specific antibodies (ELISA units) present in a 1:100,000 dilution of pooled sera from mice vaccinated with SpeA L42R, SpeA-B fusion or adjuvant only.

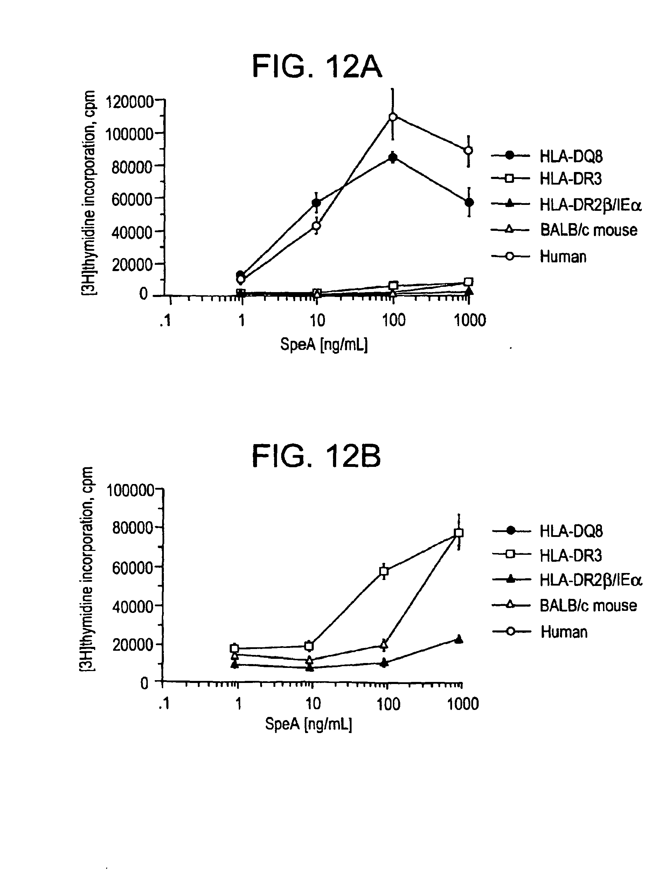

**Figure 12****.** T-cell response in vitro of mononuclear cells from transgenic mice expressing HLA-DQ8αβ and human CD4 closely approximate the physiological response of humans. Mononuclear cells were isolated from spleens of transgenic mice expressing HLA-DR3, HLA-DQ8 or HLA-DR2β/IEα, or non-transgenic BALB/c mice and human peripheral blood (1 x 10⁵/well). Following 60 h culture with SpeA, cells were pulse-labeled (12 h) with 1 uCi of [³H] thymidine. DNA from cells was harvested onto fiberglass filters and incorporated radioactivity measured by liquid scintillation.

### DETAILED DESCRIPTION

The present invention relates in part to a vaccine against superantigen toxin-associated bacterial diseases. The superantigen vaccines used in this study were developed by engineering changes in the receptor-binding portions of superantigen toxins to reduce receptor-binding affinities and toxicity while maintaining antigenicity.

Five different superantigen vaccines are described in this application: staphylococcal enterotoxin A, staplylococcal enterotoxin B, staphylococcal enterotoxin C1, toxic-shock syndrome toxin-1, and streptococcal pyrogenic exotoxin-A. For each of the superantigen toxins above, a comprehensive study of the relationships of the toxin protein structure to receptor binding was undertaken to provide insight into the design of the vaccine. The study employed site-directed mutagenesis of toxin and receptor molecules, molecular modeling, protein structure and binding studies. Following these studies, toxins were altered by site-directed mutagenesis to attenuate MHC class II binding and biological activity to an essentially non-specific level. The engineered vaccines were evaluated at each stage of analysis to determine mouse and human T-cell reactivities in vitro, serological responses and toxicity in mice and monkeys.

In one embodiment, the present invention relates to an altered superantigen toxin protein having an amino acid sequence which has been altered such that the binding of the toxin to the MHC class II receptor is disrupted.

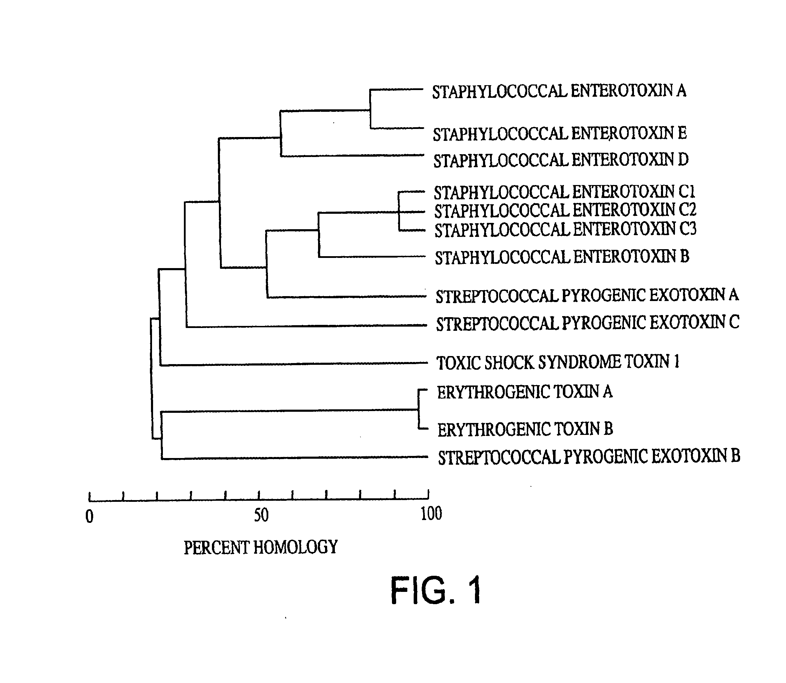

Comparison of amino acid sequences (**Fig. 1**) suggested that bacterial superantigens fall into groups consisting of (1) SEA, SED and SEE, (2) SEB, staphylococcal enterotoxins C1-C3 (SEC1-3), the streptococcal pyrogenic exotoxins A (SPE-A) and C (SPE-C), (3) TSST-1 and (4) the exfoliative toxins (ETA, ETB) and streptococcal pyrogenic exotoxin B (SPE-B), which are the most distant from the others in sequence. Although not available to the inventor when the inventions were first conceived and proof of principle was obtained, the x-ray crystallographic structures of several bacterial superantigens are now known. Diverse superantigens, such as SEB and TSST-1, appear to have little sequence in common, yet they exhibit homologous protein folds composed largely of β strands [Prasad, G.S. et al. (1993) Biochemistry 32, 13761-13766; Acharya, R.K. et al. (1994) Nature 367, 94-97; Swaminathan, S. et al. (1992) Nature 359, 801-806]within two distinct domains. Differences between the proteins are located primarily in highly variable regions comprised of several surface loops, such as the disulfide-bonded loop which is absent from TSST-1 and at the amino terminus.

The X-ray crystal structures of SEB and TSST-1 complexed with HLA DR1 are known [Kim, J. et al. (1994) Science 266, 1870-1874 ; Jardetzky, T.S. et al. (1994) Nature 368, 711-718]. The region of HLA DR1 that contacts SEB consists exclusively of α subunit surfaces. The main regions of SEB involved are two conserved sites: a polar pocket derived from three β strands of the β barrel domain and a highly solvent-exposed hydrophobic reverse turn. The polar binding pocket of SEB contains a glutamate and two tyrosines that accommodate Lys39 of the α subunit of HLA DR1, while the hydrophobic region consists of a leucine and flanking residues that make several contacts with the HLA DRα chain. The HLA DR1 binding sites of both TSST-1 and SEB overlap significantly. The hydrophobic binding contacts of other SAg with the HLA DRα chain have been proposed [Ulrich, et al. (1995). Nature, Struct. Biol 2, 554-560] to be similar to those found in SEB and TSST-1. A motif consisting of a leucine in a reverse turn [Ulrich *et al.* (1995), supra] is conserved among bacterial superantigens and may provide the key determinant (hydrophobic or otherwise) for binding HLA-DR. However, TSST-1 does not have a highly charged residue in the polar pocket that interacts with Lys39 of the HLA DRα chain and uses an alternative conformational binding mode that allows TSST-1 to interact with HLA DR1 β-chain residues and the carboxy-terminal region of the antigenic peptide.

Both SEA and SEE bind to the β subunit of DR by means of a single zinc atom [Fraser, J.D. et al. (1992) Proc. Natal. Acad. Sci. USA 89, 5507-5511]. The amino-terminal domain of SEA interfaces with the HLA DRα chain [Ulrich, *et al.* (1995)], while SEA C-terminal domain residues His187, His225 and Asp227 form a zinc-coordination complex, likely with His-81 from the β chain of an adjoining HLA DR molecule. However, our results have shown that binding of superantigen to the HLA DRβ subunit does not directly stimulate T cells [Ulrich et al. (1995) Nature, Struct. Biol. 2, 554-560], but increases the potential of the bound SEA to interact with the α chain of another HLA DR, thus increasing the biological potency.

A least-squares superimposition of the unbound molecules of modeled SEA and the crystal structure of SEB, aligned according to their structurally conserved α-helical and β-strand regions, exhibited a global folding pattern which is very similar. Differences between the two structures are calculated to be located primarily in loops of low sequence homologies, with the largest positional deviations occurring between structurally conserved regions of residues 18-20, 30-32, 173-181, 191-194, and the cysteine-loop region (90-111). Only one of these regions in SEB makes significant contact (residue Y94 [Y=tyrosine] in particular) with the HLA-DR1 molecule [Jardetzky, T.S. et al. (1994) Nature 368, 711-718].

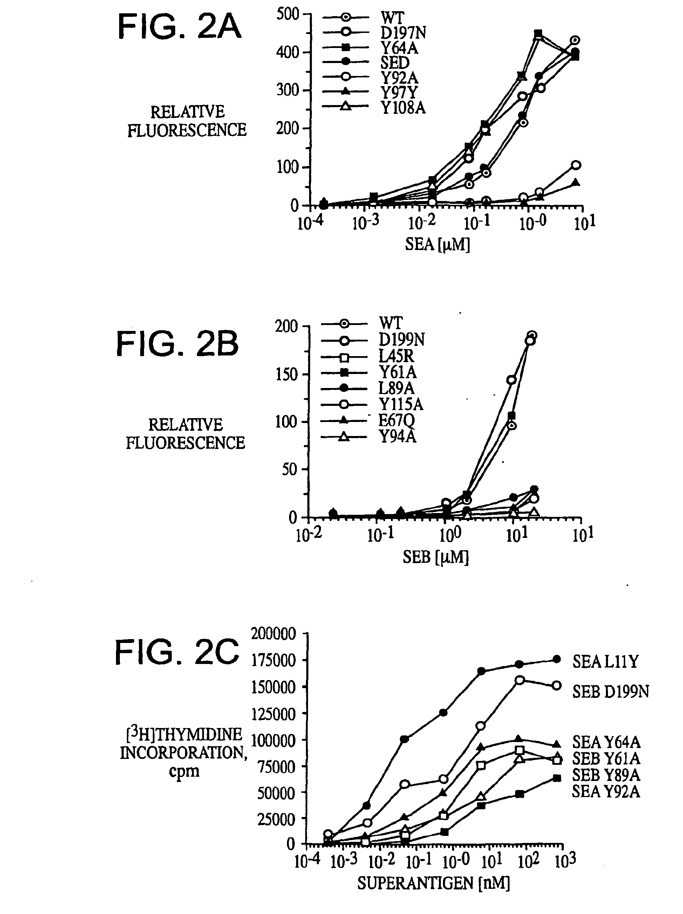

The binding interface between SEB and HLA-DR1 consists principally of two structurally conserved surfaces located in the N-terminal domain: a polar binding pocket derived from three β-strand elements of the β-barrel domain and a hydrophobic reverse turn. The binding pocket of SEB contains residues E67 (E=Glutamic acid), Y89 (Y=Tyrosine) and Y115 (Y=tyrosine), and binds K39 (K=Lysine) of the DRα subunit. The amino acid one letter code is defined as the following: A= Alanine (Ala), I= Isoleucine (Ile), L= Leucine (Leu), M= Methionine (Met), F= Phenylalanine (Phe), P= Proline (Pro), W=Tryptophan (Trp), V=Valine (Val), N= Asparagine (Asn), C=Cysteine (Cys), Q= Glutamine (Q), G= Glycine (Gly), S= Serine (Ser), T= Threonine (Thr), Y= Tyrosine (Tyr), R= Arginine (Arg), H=Histidine (His), K= Lysine (Lys), D= Aspartic acid (Asp), and E= Glutamic acid (Glu). For SEA, the binding interface with the DR molecule is modeled to contain a similar binding pocket consisting of residues D70, Y92 and Y108. Mutation of residue Y89 in SEB or Y92 in SEA to alanine (**Fig. 2**) resulted in greater than 100-fold reduction in DR1 binding. The substitution of alanine for Y89 in SEB and Y92 in SEA eliminates the hydrogen bond with K39 and disrupts packing interactions with adjacent protein residues. Modeling of the SEA mutant Y92A predicts an increase in solvent-accessible surface area for Y108 by a factor of two greater than the wild-type structure, allowing the formation of a hydrogen bond to the carboxylate group of D70 and thus disrupting key anchoring and recognition points for HLA-DR1. This effect is expected to be somewhat less in SEB due to the longer side chain at E67. Substitution of SEB Y115 with alanine also resulted in greater than 100-fold reduction of binding. In contrast, the same replacement of Y108 in SEA yielded little to no change in DR1 binding (**Fig. 2a**), suggesting the primary importance of SEA residues Y92 and D70 for stabilizing interactions with K39. The K39 side chain of DRα forms a strong.ion-pair interaction with the SEB E67 carboxylate group and hydrogen bonds with the hydroxyl groups of Y89 and Y115. Substitution of SEB E67 by glutamine reduced binding affinity by greater than 100-fold (**Fig. 2**), reflecting the replacement of the strong ionic bond with a weaker hydrogen bond. To optimize ion-pair interactions of the analogous SEA site, the shorter carboxylate side chain of D70 is predicted to shift K39 of DRα, weakening interactions with SEA Y108. The substitution of alanine for SEA Y108 is thus more easily accommodated than the homologous substitution of SEB Y115, without loss in DR1 binding.

Comparisons of the polar pocket with other bacterial superantigens were then made. SEC1-3 and SPE-A have conserved the critical DR1 binding-interface residues (**Fig. 1**), and share with SEB and SEA secondary structural elements of the DR1-binding surfaces. Asparagine in SED (N70) replaces the acidic side chain present in SEA, SEB, SPE-A and SEC1-3. Accordingly, for SED the salt bridge of the polar pocket is likely to be replaced by a hydrogen bond. Overall, DR1 affinities for SED and SEA appeared to be equivalent (**Fig. 2b**), indicating that other interactions may compensate for the absence in SED of the ion-pair found in the other superantigens. For the case of TSST-1, mutating DRα residues K39 to serine or M36 to isoleucine has been shown to greatly reduce binding [Panina-Bordignon et al. (1992) J. Exp. Med. 176: 1779-1784]. Although primarily hydrophobic, the critical TSST-1 structural elements are conserved with the SEA and SEB polar binding pocket. SEB residues Y89 and Y115 are homologous to T69 and I85 in TSST-1, respectively, and SEB E67 is replaced by I46. These TSST-1 residues are positioned in a conserved ß-barrel domain found in both SEB and SEA. However, the TSST-1 site lacks polarity equivalent to SEB/SEA, and hydrogen bonding with the hydroxyl of TSST-1 residue T69 would require that DRα K39 extend 5 Å into the pocket. TSST-1 binding utilizes an alternative strategy [Kim et al. (1994) Science 266:1870-1874] consisting of hydrophobic contacts centered around residue I46, and potential ionic or hydrogen bonds bridging DRα residues E71 and K67 to R34 and D27, respectively, of TSST-1.

The hydrophobic region of the binding interface between SEB and the HLA-DR1 molecule consists of SEB residues 44-47, located in a large reverse turn connecting β-strands 1 and 2 of SEB. These residues appear to make strong electrostatic interactions with DRα through their backbone atoms. The mutation of L45 to an arginine reduced overall HLA-DR1 binding greater than 100-fold (**Fig. 2b**), attributable to the less energetically favorable insertion of a highly charged residue into a hydrophobic depression on the DR1 molecule. The modeled DR1-SEA complex presents similar interactions with the SEA backbone atoms, with the exception of a glutamine (Q49) replacing SEB Y46. Mutation of L48 to glycine in SEA (homologous to L45 of SEB) has been reported to decrease T-cell responses. SEB L45. and the comparable L30 of TSST-1 are the most extensively buried residues in the DR1 interface. The leucine is conserved among the bacterial superantigens (**Fig. 3**) and may provide the necessary hydrophobic structural element for surface complementarity with DR1, consistent with the mutagenesis data for SEB and SEA.

The inventor has performed similar structure and function studies with TSST-1, SEC1 and SPE-A.

In determining the overall affinity of the superantigen for DR1, a contributory role is played by structural variations around the common binding motifs. A short, variable structured, disulfide-bonded loop is found in SEA and a homologous longer loop in SEB. The SEB residue Y94, contained within this loop, forms hydrophobic interactions with L60 and A61 of the DRα subunit. Replacement of Y94 with alanine partially inhibits DR1 binding (**Fig. 2a****,b**). An alanine is found in SEA (A97) and SEE at the position equivalent to SEB Y94, and mutating this residue in SEA to tyrosine results in disrupted instead of stabilized interactions with DR1 (**Fig. 2a**). Although the disulfide loops differ in structure between SEA and SEB, A97 apparently contributes to the DRα binding interface in a manner similar to Y94 of SEB. Because TSST-1 lacks a disulfide loop, similar contacts with DRα are replaced by interactions with β-strands of TSST-1. In a like manner, the absence of a salt bridge between the residues K39 of DRα and N65 of SED is apparently compensated for by stabilizing interactions occurring outside of the otherwise conserved dominant binding surfaces (**Fig. 2a**).

The amino acid residues in contact with TCR are located in regions of high sequence variability, presenting a unique surface for interaction with the TCR. Residues implicated in TCR interactions by mutagenesis of SEA and SEB reside in variable loop regions, while TSST-1 mutants that affect TCR binding are mainly located in an α helix [Acharya, R.K. et al. (1994) Nature 367, 94-97; Kim, J. et al. (1994) Science 266, 1870-1874]. Specifically, mutations that diminish T-cell receptor recognition of SEB include residues N23, Y61, and the homologous SEA N25 or Y64 (**Fig. 2c**). SEA residues S206 and N207 also control T-cell responses [Hudson, et al. (1992) J. Exp. Med. 177: 175-184]. Mutants of the polar binding pocket, SEA Y92A and SEB Y89A, equivalently reduced T-cell responses (**Fig. 2c**), reflecting the observed decreases in DR1-binding (**Fig. 2a****, b**). While supporting reduced T-cell responses, mutants SEA Y64A and SEB Y61A retained normal affinities for DR1 (**Fig. 2a****-c**).

In view of the detailed description of the present invention and the results of molecular modelling and structural studies of staphylococcal and streptococcal superantigen toxins discussed above, any amino acid sequence derived from a superantigen toxin can be altered. Sequences of several superantigen toxins are already known and available to the public in sequence databases such as GenBank, for example. The superantigen toxin sequence is preferably altered at the hydrophobic loop or polar binding pocket depending on the superantigen. Alternatively, residues adjacent to the hydrophobic loop or polar binding pocket that contact HLA-DR or residues at sites that can indirectly alter the structure of the hydrophobic loop or polar pocket can be altered. The number of residues which can be altered can vary, preferably the number can be 1-2, more preferably 2-3, and most preferably 3-4, or more with the limitation being the ability to analyze by computational methods the consequences of introducing such mutations. The residues which can be altered can be within 5 amino acid residues of the central Leucine of the hydrophobic loop (such as L45 of SEB), or within 5 residues of one of the amino acid residues of the polar binding pocket that can contact HLA-DR, (such as E67, Y89, or Y115 of SEB), more preferably, within 3 amino acid residues of the central Leucine of the hydrophobic loop (such as L45 of SEB), or within 3 residues of one of the amino acid residues of the polar pocket that can contact HLA-DR, (such as E67, Y89, or Y115 of SEB), and most preferably, the central Leucine of the hydrophobic loop (such as L45 of SEB), or one of the amino acid residues of the polar binding pocket that can contact HLA-DR, (such as E67, Y89, or Y115 of SEB). The residues can be changed or substituted to alanine for minimal disruption of protein structure, more preferably to a residue of opposite chemical characterisitcs, such as hydrophobic to hydrophilic, acidic to neutral amide, most preferably by introduction of a residue with a large hydrated side chain such as Arginine or Lysine. In addition, side chains of certain nonconserved receptor-binding surfaces, can also be altered when designing superantigen toxins with low binding affinities. These residues can include Y94 of SEB and structurally equivalent residues of other superantigens, such as A97 of SEA, or any side chain within 5 residues from these positions or any side chain in discontinuous positions (discontinuous positions are defined as amino acid residues that fold together to form part of a discrete three-dimensional structural unit but are not present on the same secondary structural unit e.g. α helix or β-strand) such as disulfide-bonded side chains, that involve, directly or indirectly, the nonconserved receptor contact surfaces outside of the polar binding pocket or hydrophobic loop. Further, amino acid residues involved with protein folding or packing can be altered when designing superantigen toxins with low binding affinities [Sundstrom et al. (1996) EMBO J. 15, 6832-6840; Sundstrom et al. (1996) J. Biol. Chem. 271, 32212-32216; Acharya et al. (1994) Nature 367, 94-97; Prasad et al. (1993) Biochem. 32, 13761-13766; Swaminathan et al. (1992) Nature 359, 801-806]. Furthermore, especially for superantigens with higher affinities for T-cell antigen receptors, side chains of amino acids within 5 residues of the position represented by N23 (conserved residue in most superantigens), N60 (conserved Asn or Trp in most superantigens) Y91 (semiconserved hydrophobic residues Trp, Ile, Val, His in most superantigens) and D210 of SEB (conserved Asp in most superantigens) can be altered when designing superantigen toxins with low binding affinities. These residues are likely to form part of the integral molecular surfaces that are in contact with T-cell antigen receptors. Because the T-cell receptor contact areas of superantigen toxins are essential for causing specific activation or inactivation of T-cell subsets, altering residues that are unique to each superantigen but that are located within 5 residues of the positions represented by N23, N60 and Y91 can produce superantigens that affect a smaller number (e.g. 1-3) of subsets. Such altered superantigen toxins can be useful as therapeutic agents.

In another embodiment, the present invention relates to a DNA or cDNA segment which encodes a superantigen toxin such as SEA, SEB, SEC-1, SpeA, and TSST-1 to name a few, the sequence of which has been altered as described above to produce a toxin protein with altered binding ability to MHC Class II and/or T-cell receptors. For SEA, the following three mutations were introduced into the toxin molecule: Tyrosine at amino acid position 92 changed to alanine; Aspartic acid at amino acid position 70 changed to arginine; Leucine at amino acid position 48 changed to arginine. The reduction in binding to HLA DR is additive per mutation, though one or two mutations can produce a vaccine and a combination of all three mutations in one molecule produces a better vaccine. Other substitutions can also result in reduced binding.

The B899445 vaccine consists of the following three mutations simultaneously introduced into the toxin molecule: tyrosine at amino acid position 89 changed to alanine; tyrosine at amino acid position 94 changed to alanine; leucine at amino acid position 45 changed to arginine. The altered superantigen toxins can be expressed either as a full-length propolypeptide or as a polypeptide in which the leader peptide has been deleted. The full-length expressed product (SEA vaccine, A489270P; SEB vaccine B899445P, B2360210P) is secreted into the periplasmic space of E. *coli* host cells, and the leader peptide is recognized and cleaved by a native bacterial enzymatic mechanism. The altered superantigen toxins in which the leader peptide has been deleted (A489270C, B899445C), the first residue of the mature protein is encoded by the transcriptional start site and codon for methionine (ATG), and the protein is expressed as a nonsecreted product within the host E. *coli* cell. For the TSST-1 vaccine TST30, the leucine at position 30 was changed to arginine. For the SEC1 vaccine, SEC45, the leucine at position 45 was changed to arginine. For the SPE-A vaccine, SPEA42, the leucine at position 42 was changed to arginine.

In another embodiment, the present invention relates to a recombinant DNA molecule that includes a vector and a DNA sequence as described above. The vector can take the form of a plasmid such as any broad host range expression vector for example pUC18/19, pSE380, pHIL, pET21/24 and others known in the art. The DNA sequence is preferably functionally linked to a promoter such that the gene is expressed when present in an expression system and an altered superantigen toxin is produced. The expression system can be an in vitro expression system or host cells such as prokaryotic cells, or *in vivo* such as DNA vaccines.

In a further embodiment, the present invention relates to host cells stably or transiently transformed or transfected with the above-described recombinant DNA constructs. The host can be any eukaryotic or prokaryotic cell including but not limited in E. coli DH5α or BL21. The vector containing the altered superantigen toxin gene is expressed in the host cell and the product of the altered toxin gene, whether a secreted mature protein or a cytoplasmic product, can be used as a vaccine or as a reagent in diagnostic assays or detection methods, or for therapeutic purposes. Please see e.g., Maniatis, Fitsch and Sambrook, Molecular Cloning: A Laboratory Manual (1982) or DNA Cloning, Volumes I and II (D. N. Glover ed. 1985) for general cloning methods. The DNA sequence can be present in the vector operably linked to a highly purified IgG molecule, an adjuvant, a carrier, or an agent for aid in purification of altered toxin. The transformed or transfected host cells can be used as a source of DNA sequences described above. When the recombinant molecule takes the form of an expression system, the transformed or transfected cells can be used as a source of the altered toxin described above.

A recombinant or derived altered superantigen toxin is not necessarily translated from a designated nucleic acid sequence; it may be generated in any manner, including for example, chemical synthesis, or expression of a recombinant expression system. In addition the altered toxin can be fused to other proteins or polypeptides for directing transport for example into the periplasm or for secretion from the cell. This includes fusion of the recombinant or derived altered superantigen to other vaccines or sequences designed to aid in purification, such as His-tagged, epitope-tagged or antibody Fc-fusions.

In a further embodiment, the present invention relates to a method of producing altered superantigen toxin which includes culturing the above-described host cells, under conditions such that the DNA fragment is expressed and a superantigen toxin protein is produced. The superantigen toxin can then be isolated and purified using methodology well known in the art such as immunoaffinity chromatography or preparative isoelectric focusing. However, the method of purification is not critical to the performance of the vaccine. The altered superantigen toxin can be used as a vaccine for immunity against infection with bacterial superantigen toxins or as a diagnostic tool for detection of superantigen toxin-associated disease or bacterial infection. The transformed host cells can be used to analyze the effectiveness of drugs and agents which affect the binding of superantigens to MHC class II or T-cell antigen receptors. Chemically derived agents, host proteins or other proteins which result in the down-regulation or alteration of expression of superantigen toxins or affect the binding affinity of superantigen toxins to their receptors can be detected and analyzed. A method for testing the effectiveness of a drug or agent capable of altering the binding of superantigen toxins to their receptors can be for example computer-aided rational design or combinatorial library screening, such as phage display technology.

In another embodiment, the present invention relates to antibodies specific for the above-described altered superantigen toxins. For instance, an antibody can be raised against the complete toxin or against a portion thereof. Persons with ordinary skill in the art using standard methodology can raise monoclonal and polyclonal antibodies to the altered superantigens of the present invention, or a unique portion of the altered superantigen. Materials and methods for producing antibodies are well known in the art (see for example Goding, in, Monoclonal Antibodies: Principles and Practice, Chapter 4, 1986). The antibodies can be used in diagnostic assays for detection of superantigen toxin-associated infection. Neutralizing antibodies can be used in a therapeutic composition for the treatment of amelioration of anergy and/or for the treatment of a superantigen toxin-associated infection.

In a further embodiment, the present invention relates to a method for detecting the presence of superantigen-associated bacterial infections in a sample. Using standard methodology well known in the art, a diagnostic assay can be constructed by coating on a surface (i.e. a solid support) for example, a microtitration plate or a membrane (e.g. nitrocellulose membrane), all or a unique portion of the altered superantigen described above, and contacting it with the serum of a person suspected of having a superantigen-associated bacterial infection. The presence of a resulting complex formed between the altered superantigen toxin and antibodies specific therefor in the serum can be detected by any of the known methods common in the art, such as fluorescent antibody spectroscopy or colorimetry. This method of detection can be used, for example, for the diagnosis of superantigen-associated bacterial infections.

In yet another embodiment, the present invention relates to a method for detecting the presence of superantigen toxin in a sample. Using standard methodology well known in the art, a diagnostic assay can be constructed by coating on a surface (i.e. a solid support) for example, a microtitration plate or a membrane (e.g. nitrocellulose membrane), antibodies specific for altered superantigen toxin, and contacting it with serum or tissue sample of a person suspected of having superantigen-associated bacterial infection. The presence of a resulting complex formed between toxin in the serum and antibodies specific therefor can be detected by any of the known methods common in the art, such as fluorescent antibody spectroscopy or colorimetry. This method of detection can be used, for example, for the diagnosis of superantigen-associated bacterial infection or disease such as food poisoning and toxic-shock syndrome or the detection of superantigen toxin in food and drink.

In another embodiment, the present invention relates to a diagnostic kit which contains altered superantigen toxin from a specific bacteria or several different superantigen toxins from bacteria and ancillary reagents that are well known in the art and that are suitable for use in detecting the presence of antibodies to superantigen toxin-associated bacteria in serum or a tissue sample. Tissue samples contemplated can be avian, fish, or mammal including monkey and human.

In yet another embodiment, the present invention relates to a vaccine for protection against superantigen toxin-associated bacterial infections. The vaccine can comprise one or a mixture of individual altered superantigen toxins, or a portion thereof. When a mixture of two or more different altered superantigen toxin from different bacteria is used, the vaccine is referred to as a multivalent bacterial superantigen vaccine. The vaccine is designed to protect against the pathologies resulting from exposure to one or several related staphylococcal and streptococcal toxins. In addition, the protein or polypeptide can be fused or absorbed to other proteins or polypeptides which increase its antigenicity, thereby producing higher titers of neutralizing antibody when used as a vaccine. Examples of such proteins or polypeptides include any adjuvants or carriers safe for human use, such as aluminum hydroxide.

The staphylococcal enterotoxin (SE) serotypes SEA, SED, and SEE are closely related by amino acid sequence, while SEB, SEC1, SEC2, SEC3, and the streptococcal pyrogenic exotoxins B share key amino acid residues with the other toxins, but exhibit only weak sequence homology overall. However, there are considerable similarities in the known three-dimensional structures of SEA, SEB, SEC1, SEC3, and TSST-1. Because of this structural similarity, it is likely that polyclonal antibodies obtained from mice immunized with each SE or TSST-1 exhibit a low to high degree of cross-reaction. In the mouse, these antibody cross-reactions are sufficient to neutralize the toxicity of most other SE/TSST-1, depending upon the challenge dose. For example, immunization with a mixture of SEA, SEB, TSST-1 and SpeA was sufficient to provide antibody protection from a challenge with any of the component toxins, singly or in combination.

The likelihood of substantial antigen-cross-reactivity suggests that it may be possible to obtain immune protection for other (or perhaps all) staphylococcal superantigens by use of a minimal mixed composition of vaccines. For the case of staphylococcal superantigens, a combination of the component vaccines from SEA, SEB, SEC-1 and TSST-1 should be sufficient to provide immune protection against SEA, SEB, SEC1-3, and TSST-1. The addition of SpeA component to the trivalent mixture will allow for sufficient protection against the streptococcal toxins SpeA and SPEc. Therefore, a multivalent vaccine consisting of the altered superantigen toxins from SEA, SEB, SEC-1, TSST-1, and SpeA as described above, is predicted to provide protective immunity against the majority of bacterial superantigen toxins.

The vaccine can be prepared by inducing expression of a recombinant expression vector comprising the gene for the altered toxin described above. The purified solution is prepared for administration to mammals by methods known in the art, which can include filtering to sterilize the solution, diluting the solution, adding an adjuvant and stabilizing the solution. The vaccine can be lyophilized to produce a vaccine against superantigen toxin-associated bacteria in a dried form for ease in transportation and storage. Further, the vaccine may be prepared in the form of a mixed vaccine which contains the altered superantigen toxin(s) described above and at least one other antigen as long as the added antigen does not interfere with the effectiveness of the vaccine and the side effects and adverse reactions, if any, are not increased additively or synergistically. Furthermore, the vaccine may be administered by a bacterial delivery system and displayed by a recombinant host cell such as *Salmonella* spp, *Shigella* spp, *Streptococcus* spp. Methods for introducing recombinant vectors into host cells and introducing host cells as a DNA delivery system are known in the art [Harokopakis et al. (1997) Infect. Immun. 65, 1445-1454; Anderson et al. (1996) Vaccine 14, 1384-1390; Medaglini et al. (1995) Proc. Natl. Acad. Sci. U.S.A. 92, 6868-6872].

The vaccine may be stored in a sealed vial, ampule or the like. The present vaccine can generally be administered in the form of a liquid or suspension. In the case where the vaccine is in a dried form, the vaccine is dissolved or suspended in sterilized distilled water before administration. Generally, the vaccine may be administered orally, subcutaneously, intradermally or intramuscularly but preferably intranasally in a dose effective for the production of neutralizing antibody and protection from infection or disease.

In another embodiment, the present invention relates to a method of reducing superantigen-associated bacterial infection symptoms in a patient by administering to said patient an effective amount of anti-altered superantigen toxin antibodies, as described above. When providing a patient with anti-superantigen toxin antibodies, or agents capable of inhibiting superantigen function to a recipient patient, the dosage of administered agent will vary depending upon such factors as the patient's age, weight, height, sex, general medical condition, previous medical history, etc. In general, it is desirable to provide the recipient with a dosage of the above compounds which is in the range of from about 1pg/kg to 10 mg/kg (body weight of patient), although a lower or higher dosage may be administered.

In a further embodiment, the present invention relates to a therapeutic method for the treatment of diseases that may not be associated directly with superantigen toxins but which result in specific nonresponsiveness of T-cell subsets or detection of abnormally low level of subsets in peripheral blood, said method comprising the administration of altered superantigen toxins, *in vivo* or *ex vivo*, such that T-cell subsets are expanded or stimulated. Diseases which cause anergy or nonresponsiveness of T-cells include, but are not limited to, infectious diseases and cancers. The desired clinical outcome such as an increase in detectable T cell subsets or in stimulation *ex vivo* of T-cells through their antigen receptors, such as by antigen or anti-CD3 antibody can be measured by standard clinical immunology laboratory assays.

In yet another embodiment, the present invention relates to a therapeutic method for the treatment of diseases associated with expanded or over-stimulated T-cell subsets, such as autoimmunity for example, said method comprising administration *in vivo* or *ex vivo*, of superantigen toxin altered in such a manner that only limited (1-3) T-cell subsets are stimulated but that MHC class II binding affinity still remains, such that anergy or inactivation of T-cells is produced. The desired clinical outcome can be measured as a reduction of circulating blood T-cells of the targeted subset(s) or diminished antigen or other antigen receptor-mediated-stimulatory responses by assays known in the art.

Described below are examples of the present invention which are provided only for illustrative purposes, and not to limit the scope of the present invention. In light of the present disclosure, numerous embodiments within the scopy of the claims will be apparent to those of ordinary skill in the art.

The following Materials and Methods were used in the Examples that follow.

### Structural comparisons

Primary protein structure data are available for several bacterial superantigens, including SEA, SED, SEB, SEC1-3, TSST-1. Superantigens for which structures were unavailable were modeled using comparative techniques (HOMOLOGY program; Biosym Technologies, Inc., San Diego, CA). Before x-ray crystallography data was available, SEA was modeled by using this method, and the model was in very close agreement with the experimentally determined structure. As an example, the amino acid sequence for SEA was aligned with the known structure of free and HLA-DR1 bound SEB, and the SEA molecule was built for both free and DR1-bound proteins. Loop segments of SEA were generated by a de novo method. Refinement of the modeled structures was carried out by means of molecular-dynamics simulations (DISCOVER, Biosym). The constructed free SEA molecule was immersed in a 5-Å layer of solvent water and the α-carbon atoms lying in the structurally conserved regions were tethered to their initial positions during the simulations. For the bound SEA molecule, simulations were carried out by constructing an active-site region composed of part of the SEA molecule and the DR1 molecule inside a 10-Å interface boundary, as derived from the crystal structure of the DR1-SEB complex. Amino acid residues lying in the outer boundary were rigidly restrained at their initial positions. The active-site region was immersed in a 5-Å layer of water. Protein interactions were modeled by employing the consistent valence force field with a non-bonded cutoff distance of 11.0 Å. Simulations were initiated with 100 cycles of minimization using a steepest descent algorithm followed by 100-ps relaxation (using a 1.0 fs timestep). Structural comparisons between SEB, SEC1, and TSST-1 were performed by using the crystal structures (Brookhaven data holdings) aligned according to common secondary structural elements and/or by sequence and structural homology modeling.

### Site-specific mutagenesis

Site-specific mutagenesis was performed according to the method developed by Kunkel, using gene templates isolated from Staphylococcus aureus strains expressing SEA (FDA196E, a clinical isolate, Fraser, J.D. (1994) Nature 368: 711-718), SEB (14458, clinical isolate), SEC1 (Toxin Technologies, Sarasota, FL), TSST-1 (pRN6550 cloned product, a clinical isolate, Kreiswirth, B. N. et al. (1987) Mol. Gen. Genet. 208, 84-87), and SpeA (Toxin Technologies), respectively. Modified T7 polymerase (Sequenase, U.S. Biochemical Corp., Cleveland, OH) was used to synthesize second-strand DNA from synthetic oligonucleotides harboring the altered codon and single-stranded, uracil-enriched M13 templates. Mutagenized DNA was selected by transforming E. *coli* strain JM101. Alternatively, double stranded DNA was used as template for mutagenesis. Mutagenized sequences were confirmed by DNA sequencing (Sanger et al., 1977, Proc. Natl. Acad. Sci. USA 74: 5463-5467; Sambrook *et al.*, 1989) using synthetic primers derived from known sequences, or universal primers. The complete coding sequences were inserted into expression plasmids such as pUC19, pSE380 or pET21 for production in E. *coli* hosts.

### Protein purifications

The appropriate E. *coli* hosts were transformed with plasmids harboring the mutant toxin genes. In general, the bacteria were grown to an A600 0.5-0.6 in Terrific Broth (Difco Laboratories, Detroit, MI) containing 50 µg/mL ampicillin or kanamycin. Recombinant proteins were induced with isopropyl-β-D-thio-galactopyranoside (Life Technologies, Gaithersburg, MD) and recovered as cytoplasmic or bacterial periplasmic secretion products. Bacteria were collected by centrifugation, washed with 30 mM NaCl, 10 mM TRIS (pH 7.6), and pelleted by centrifugation and either lysed or osmotically shocked for collection of secreted proteins. Preparations were isolated by CM Sepharose ion-exchange chromatography, rabbit antibody (Toxin Technologies, Sarasota, FL) affinity columns, ion exchange HPLC or similar methods. In some cases partially purified superantigen was further purified by preparative isoelectric focusing (MinipHor; Rainin Instrument Company, Inc., Woburn, MA.). The MinipHor was loaded with the SEA-enriched fraction from CM Sepharose chromatography in a solution containing 10% (v/v) glycerol and 1% (v/v) pH 6-8 ampholytes (Protein Technologies, Inc., Tucson, AZ). The protein preparations were allowed to focus until equilibrium was reached (approximately 4 hr, 4°C). Twenty focused fractions were collected and aliquots of each were analyzed by SDS-polyacrylamide gel electrophoresis (SDS-PAGE) and immunoblotting. The SEA-containing fractions were pooled, and refocused for an additional 4 h. The fractions containing purified SEA were pooled and dialyzed first against 1 M NaCl (48 h, 4°C) to remove ampholytes, and then against PBS (12 h, 4°C). Legitimate amino-terminal residues were confirmed by protein sequencing. Precise measurements of protein concentrations were performed by immunoassay using rabbit antibody affinity-purified with the wild-type superantigens and by the bicinchoninic acid method (Pierce, Rockford, IL) using wild-type protein as standards. All protein preparations were >99% pure, as judged by SDS-PAGE and Western immunoblots. In some cases, as when used for lymphocyte assays, bacterial pyrogens were removed by passing the protein preparations over Polymyxin B affinity columns.

### Binding of superantigens to HLA-DR1

The DR1 homozygous, human B-lymphoblastoid cell line LG2 or L cells transfected with plasmids encoding HLA-DR1αβ were used in the binding experiments. Cells were incubated 40 min (37°C) with wild-type or mutant superantigen in Hanks balanced salt solution (HBSS) containing 0.5% bovine serum albumin. The cells were washed with HBSS and then incubated with 5 µg of specific rabbit antibody (Toxin Technology, Sarasota, FL) for 1 h on ice. Unbound antibody was removed, and the cells were incubated with FITC-labelled goat anti-rabbit IgG (Organon Teknika Corp., Durham, N.C.) on ice for 30 min. The cells were washed and analyzed by flow cytometry (FACScan; Becton Dikinson & Co., Mountain View, CA). Controls consisted of cells incubated with affinity purified anti-toxin and the FITC labelled antibody without prior addition of superantigen.

### Lymphocyte proliferation

Human peripheral blood mononuclear cells were purified by Ficoll-hypaque (Sigma, St. Louis, MO) buoyant density gradient centrifugation. Genes encoding the human MHC class II molecules DR1αβ (DRA and DRB1*0101 cDNA [Bavari and Ulrich (1995) Infect. Immun. 63, 423-429] were cloned into the eukaryotic expression vector pRC/RSV (Invitrogen, Carlsbad, CA), and mouse L cells were stably transfected. The transfectants were selected by fluorescence-activated cell sorting (EPICS C, Coulter Corp., Hialeah, FL) using rabbit anti-DRαβ antisera and FITC-goat anti-rabbit IgG, to produce cells that expressed a high level of DRαβ21. 1 x 10⁵ cells/well of a 96-well plate were irradiated (15,000 Rad), and wild-type or mutant SE, was added. After a brief incubation period (45 min, 37°C), unbound SE was rinsed from the culture plates using warm media. The cells were cultured in RPMI-1640 (USAMRIID) with 5% FBS for 72 h, and pulsed-labelled for 12 h with 1µCi [³H]-thymidine (Amersham, Arlington Heights, IL). Cells were harvested onto glass fiber filters, and [³H]-thymidine incorporation into the cellular DNA was measured by a liquid scintillation counter (BetaPlate, Wallac Inc., Gaithersburg, MD). Splenic mononuclear cells or human peripheral blood mononuclear cells were obtained by buoyant density centrifugation (Histopaque; Sigma Chemical Comp.) and washed three times. The cells were resuspended in medium containing 5% fetal bovine serum (FBS), and 100 µl (4 x 10⁵ cells) of the cell suspension was added to triplicate wells of 96-well flat bottom plates. The mononuclear cells were cultured (37°C, 5% CO₂) with WT or mutant SEA. After 3 days the cultures were pulsed (12h) with 1 µCi/well of [³H] thymidine (Amersham, Arlington Heights, IL) and incorporated radioactivity was measured by liquid scintillation.

### Gel electrophoresis and immunoblotting analysis.

The protein preparations were analyzed by SDS-PAGE (12%) and stained with Coomassie Brilliant Blue R-250 (Sigma Chemical Comp. St Louis, MO) in methanol (10% v/v) acetic acid (10% v/v). The proteins separated by SDS-PAGE (not stained) were transferred to nitrocellulose membranes (Bio-Rad Lab. Inc., Melville, NY) by electroblotting, and the membranes were then blocked (12 h, 4°C) with 0.2% casein in a buffer consisting of 50 mM sodium phosphate, 140 mM sodium chloride, pH 7.4 (PBS). The membrane was then incubated (1 h, 37°C, shaking) with 2 µg/mL of affinity-purified anti-toxin antibody (Toxin Technology, Sarasota, FL) in PBS with 0.02% casein. After the membranes were thoroughly washed, peroxidase-conjugated goat anti-rabbit IgG (Cappel/Organon Teknika Corp., West Chester, PA) was added (1:5,000) and the membranes were incubated for 1 h (37°C) with shaking. The unbound antibody was removed by washing with PBS and bound antibody was visualized by using a Bio-Rad peroxidase development kit (Biorad, Hercules, CA). For quantitation, dilutions of wild-type preparations were immobilized on nitrocellulose membranes by using a Slot-Blot apparatus (Bio-Rad). The membrane was removed from the Slot-Blot apparatus and unreacted sites were blocked (12h, 4°C) with 0.2% casein in PBS. After washing once with the PBS, the membrane was incubated (1h, 37°C) with 2 µg/mL rabbit affinity purified anti-toxin antibody (Toxin Technology) in PBS that contained 0.02% casein. After four washes, the bound rabbit antibody was reacted with goat anti-rabbit IgG conjugated with horseradish peroxidase (1 h, 37°C) and the blots were developed using enhanced chemiluminescence (ECL; Amersham Life Sciences, Arlington Heights, IL) or similar methods. The amount of mutant protein was measured by densitometry (NIH Image 1.57 software, National Institutes of Health, Bethesda, MD) of exposed X-ray film. Standard curves were prepared by plotting the mean of duplicate densitometric readings for each dilution of toxin standard. The resulting values were fitted to a straight line by linear regression. Concentrations of proteins were determined by comparing mean values of various dilutions of the mutant to the standard curve.

### Biological activities and Immunizations.

Male C57BL/6 mice, 10 to 12-weeks old, were obtained from Harlan Sprague-Dawley, Inc. (Frederick Cancer Research and Development Center, Frederick, MD). The lethal effect of WT or mutant SEA was evaluated as described in Stiles et al. (1993) Infect. Immun. 61, 5333-5338. For immunizations, mice were given by interperitoneal (ip) injections either 2 or 10 µg of WT or mutant toxin in 100 µl of adjuvant (RIBI, Immunochem Research, Inc. Hamilton, MT or alum), or adjuvant only, and boosted (ip) at 2 and 4 weeks. Serum was collected from tail veins one week after the last immunization. Mice were challenged 2 weeks after the last injection with toxin and lipopolysaccharide (LPS, 150 µg) from *E. coli* 055:B5 serotype (Difco Laboratories, Detroit, MI). Challenge controls were adjuvant-immunized or non-immunized mice injected with both agents (100% lethality) or with either wild type toxin or LPS. No lethality was produced by these negative controls. Monkeys were immunized with the antigen in the right leg, caudal thigh muscles. Each received three intramuscular immunizations with a superantigen vaccine plus adjuvant. Control monkeys received 0.5 ml total volume of adjuvant (Alhydrogel, Michigan Department of Public Health) and sterile PBS using the same techniques and equipment as the immunized monkeys. Immunizations were administered 28±2 days apart and consisted of 20 µg of the vaccine in adjuvant in a total volume of 0.5 ml. Immunizations were administered on day 0, 28±2, and 56±2 using a 23-27 ga 1/2-5/8" needle attached to a 1 ml tuberculin syringe into the caudal thigh.

### Antibody assay.

Microtiter plates were coated with 1 µg/well of WT toxin in 100 µl of PBS (37°C, 2 h). After antigen coating, the wells were blocked with 250 µl of casein 0.2% in PBS for 4 h at 37°C and then washed four times with PBS containing 0.2% Tween 20. Immune or nonimmune sera were diluted in PBS containing 0.02% casein and 100 µl of each dilution was added to duplicate wells. After each well was washed four times, bound antibody was detected with horse radish peroxidase (Sigma Chemical Comp., St. Louis, MO) labelled goat anti-species specific IgG (37°C, 1 h), using O-phenylenediamine as the chromogen. Mean of duplicates OD (absorbance at 490 nm) of each treatment group was obtained and these data were compared on the basis of the inverse of the highest serum dilution that produced an OD reading four times above the negative control wells. For negative controls, antigen or serum was omitted from the wells.

### Superantigen binding and TCR subset analysis.

Cells from the mouse B-lymphoma line A20 (ATCC, Rockville, MD) (2-4 x 10⁵ cells) were incubated (40 min at 37°C) with WT or mutant toxin in Hanks balanced salt solution containing 0.5% bovine serum albumin (HBSS, USAMRIID). The cells were washed with HBSS and incubated with 5 µg of affinity-purified anti-toxin antibody in HBSS (4°C, 45 min). Unbound antibody was removed and the bound antibody was detected with fluorescein isothiocyanate (FITC)-labelled, goat anti-rabbit IgG (Organon Teknika Corp., Durham, NC). Unbound antibody was removed and the cells were analyzed by with a FACSort flow cytometer (Becton Dikinson & Co. , Mountain View, CA).

For TCR subset analysis, splenic mononuclear cells were obtained from mice immunized with WT or mutant toxin. The mononuclear cells were incubated (37°C) with WT toxin (100 ng/mL) for 5 days and then cultured in 85% RPMI-1640, 10% interleukin-2 supplement (Advanced Biotechnologies Inc., Columbia, MD) with 5% FBS for an additional 5 days. The T cells were washed twice and stained with anti-TCR (Biosource, Camarillo, CA) or anti-Vβ specific TCR (Biosource, Camarillo, CA) (45 min, 4°C). All cells analyzed were positive for T cell marker CD3+ and expressed the CD25 activation marker (data not shown). Controls were incubated with an isotype matched antibody of irrelevant specificity. Unreacted antibody was removed, and the cells were incubated with an FITC-labelled, anti-mouse IgG (Organon Teknika Corp, Durham, NC) on ice for 30 min. The cells were washed and analyzed by flow cytometry (FACSort).

### LPS potentiation of SE toxicity in mice.

C57BL/6 or BALB/c mice weighing 18-20 g (Harlan Sprague Dawley, Inc., Frederick Cancer Research and Development Center, Frederick, MD) were each injected intraperitoneally (i.p.) with 200 µl of PBS containing varying amounts of SEA, SEB, or SEC1, TSST-1, or SpeA followed 4 h later with 75 or 150 µg of LPS (200 µl/i.p.). Controls were each injected with either SE (30 mg) or LPS (150 mg). Animals were observed for 72 h after the LPS injection. Calculations of LD50 were done by Probit analysis using 95% fiducial limits (SAS Institute Inc., Cary., NC).

The biological effects of SEA and SEB were also tested in transgenic C57BL/6 mice (GenPharm International, Mountain View, CA) deficient in MHC class I or II expression [Stiles et al. (1993) Infect. Immun. 61, 5333-5338], as described above, using a single dose of toxin (30 µg/mouse). Genetic homozygozity was confirmed by Southern analysis of parental tail DNA, using β2 microglobulin and MHC class II β DNA probes.

### Detection of cytokines in serum.

Mice (n=18 per group) were injected with toxin (10 µg), LPS (150 µg), or toxin plus LPS. Sera were collected and pooled from three mice per group at each time point (2, 4, 6, 8, 10, 22 h) after LPS injection. Sera were collected at various time points following toxin injection (-4 h, or 4h before LPS injection, for data tabulation). Collection of LPS control sera began at the time of injection (0 h).

Serum levels of TNFα and IL-α were detected by an enzyme linked immunosorbent assay (ELISA). TNFα was first captured by a monoclonal antibody against mouse TNFα (GIBCO-BRL, Grand Island, NY) and then incubated with rabbit anti-mouse TNFα antibody (Genzyme, Boston, MA). The ELISA plate was washed and peroxidase conjugate of anti-rabbit antibody (Boehringer Mannheim, Indianapolis, IN) added to the wells. After washing the plate and adding substrate (Kirkegaard and Perry, Gaithersburg, MD), TNFα concentrations were measured using the mean A450 reading of duplicate samples and a standard curve generated from recombinant mouse TNFα (GIBCO-BRL). Serum levels of IL-1α were determined from the mean reading of duplicate samples with an ELISA kit that specifically detects murine IL-1α (Genzyme, Boston, MA). The standard error of the mean (SEM) for TNFα and IL-1α readings was +/- 5%.

Quantitation of IL-6 and IFNγ were measured by bioassays [See *et al.* (1990) Infect. Immun. 58: 2392-2396]. An IL-6 dependent cell line, 7TD1 (kindly provided by T. Krakauer), was used in a proliferative assay with serial two-fold dilutions of serum samples assayed in triplicate. Proliferation of 7TD1 cells in a microtiter plate was measured by uptake of [³H]-thymidine (1 µCi/well; Amersham, Arlington Heights, IL) and the activity of IL-6 from serum was compared to a recombinant mouse IL-6 standard (R and D Systems, Minneapolis, MN) as previously described [See *et al.* (1990) Infect. Immun. 58: 2392-2396]. The SEM of triplicate samples was +/- 10%.

IFNγ was measured by the reduction of vesicular stomatitis virus (New Jersey strain) cytopathic effects on L929 cells, as previously described [Torre et al. (1993) J. Infect. Dis. 167, 762-765]. Briefly, serial two-fold dilutions of serum were made in duplicate and added to microtiter wells containing L929 cells (5 x 10⁴/well). After incubating 24 h, virus (5 x 10⁵ PFU/well) was added and the cytopathic effects measured at 48 h by absorbance readings (570 nm) of reduced 3-[4, 5-dimethylthiazol-2-yl]-2,5 diphenyl tetrazolium bromide (Sigma). The activity of each serum sample was determined using recombinant mouse IFNγ as a standard (Biosource, Camarillo, CA). The SEM of duplicate samples was +/- 5%.

### Protein production.

Reagents and Solutions:
Bacterial Wash Buffer #1:10mM Tris/30mM NaCl, 10ml of 1M Tris (Sigma), pH 7.6 , 6ml of 5M NaCl (Sigma), adjust volume with H₂O to 1 Liter.
Inclusion Product Wash Buffer #2: 10mM Tris/100mM NaCl, 1 ml of 1M Tris, pH 8.0, 2 ml of 5M NaCl, adjust volume with H₂O to 100 ml
Lysis Buffer: 375 µL of 1M Tris, pH 8.0, 30µL of 500mM EDTA (Sigma), 300 µL of 5M NaCl, 15 mL final vol. Refolding Buffer: 4.8g of Urea (4M Urea final soln.; Sigma), 2ml of 1M Tris, pH 8.5, 100µL of 1M DTT (final conc. of 5 mM; Life Technologies).
DNAse: 100 U/µL, frozen aliquots, (reconstituted with Lysis Buffer; Pharmacia Biotech). Lysozyme: 10 mg/ml stock, frozen.aliquots; (reconstituted with Lysis Buffer; Sigma). DOC: (Sodium Deoxycholate); powder (Sigma). DTT: (Dithiothreitol); 1 M in H₂0, frozen aliquots (Life technologies). IPTG: (isopropyl β-D-thiogalactopyranoside; 500 mM stock;Life technologies). Kanamycin: (50 mg/ml stock in H₂0; Sigma)

A single bacterial colony from a fresh streak plate of BL21 (DE3) harboring the expression plasmid was used to innoculate a starter culture of 100 ml of media (e.g. LB or Terrific Broth), containing the appropriate antibiotic (kanamycin, 50µg/ml final or 75 µg/mL ampicillin). The culture was grown for 12-16 hours (overnight) in an incubator/shaker at 37°C. A shaker incubator with chiller/heater combination was used to provide reliable temperature control. Preparative cultures were innoculated with 10-50 ml of the fresh seed culture per 1 L of pre-warmed (37°C) media, containing antibiotic (e.g. 50 µg/ml kanamycin). Cultures (37°C) were grown and the bacterial density was monitored in 30 min intervals beginning 2 hours after innoculation. Incubator temperature was then dropped to induction temperature of 30°C. A final concentration of 1 mM IPTG was added when culture reached 1/2 log growth incubation was continuted (30°C) for an additional 2-4 hours. The bacterial cultures were transferred to 500ml Sorvall centrifuge bottles and bacteria pelleted by centrifugation in a Sorvall RC5C centrifuge (5000 rpm, 20 min, 4°C, GS3 rotor). The supernatant was discarded and pellets were held on ice (4°C). The bacterial pellets were resuspended in 400ml of Bacterial Wash Buffer #1. Pellet the bacteria by centrifugation in Sorvall RC5C centrifuge (5000 rpm, 20 min, 4°C, GS3 rotor). Supernatant was discarded the bacterial pellet resuspended in Bacterial Wash Buffer #1; 50 ml of buffer/2.5 ml of pelleted bacteria. The bacterial pellet was concentrated by centrifugation and frozen (-20°C). Bacterial pellet was rapidly thawed in a 37°C water bath, resuspended by mixing (1-2 min) pellet in 15ml Lysis Buffer. Next 400µL lysozyme (10mg/ml stock) was added and mixed for 30 min (20-22°C) on rotator.

Dry DOC (20 mg) was stirred into bacterial suspension with a clean pipette for 10 min in a 37°C water bath and 500 Units of DNAse was then added. After mixing (20-22°C) for 30 min, the lysed bacteria were transferred by pipet to a clean 50 ml high speed centrifuge tube and the inclusion granules were pelleted by centrifugation (Heraeus Sepatech rotor, Baxter Biofuge 17R table-top centrifuge, 11000 rpm, 15 min, 4°C).

The inclusions were washed 2x by centrifugation in 5ml Inclusion Product Wash Buffer #2 (Heraeus Sepatech rotor, Baxter Biofuge 17R table-top centrifuge, 11000 rpm, 15 min, 4°C), resuspended in 20ml Refolding Buffer and rotated 2 hour (20-22°C). The solution was cleared by centrifugation and nondissolved protein removed. Supernatants were dialyzed against 2L of Phosphate Buffered Saline (PBS, pH 7.4), for 12-16 hours (4°C) and any precipitated material removed by centrifugation. The cleared, PBS-equilibrated product was filter sterilized (0.45 micron filter) and frozen until use (-20°C).

Western Immunoblots. Proteins (approx. 2 ug/lane) were electrophoresed through 12% polyacrylamide gels in the presence of SDS (1%), with dithiothreitol (2 mM). Gels were then electroblotted onto a protein-binding membrane (Amersham), and blocked (2 h, 37°C) with 0.2% casein in PBS. The membrane was then incubated (1 h, 37°C) with a 1/200 dilution of affinity-purified, rabbit anti-SpeA or SpeB (Toxin Technologies, Sarasota, FL). Unbound antibody was washed from the membrane using PBS, and bound antibody was detected with peroxidase conjugated, goat anti-rabbit antisera, using a commercial color development kit (BioRad, Richmond, CA).

### EXAMPLE 1

### Molecular modeling and structural studies of staphylococcal and streptococcall superantigens: bacterial superantigens share common 3-dimensional structure.

Comparison of amino acid sequences (**Fig. 1**) suggested that bacterial superantigens fall into groups consisting of (1) SEA, SED and SEE, (2) SEB, staphylococcal enterotoxins C1-C3 (SEC1-3), the streptococcal pyrogenic exotoxins A (SPE-A) and C (SPE-C), (3) TSST-1 and (4) the exfoliative toxins (ETA, ETB) and streptococcal pyrogenic exotoxin B (SPE-B), which are the most distant from the others in sequence. Although not available to the inventor when the inventions were first conceived and proof of principle was obtained, the x-ray crystallographic structures of several bacterial superantigens are now known. Diverse superantigens, such as SEB and TSST-1, appear to have little sequence in common, yet they exhibit homologous protein folds composed largely of β strands [Prasad, G.S. et al. (1993) Biochemistry 32, 13761-13766; Acharya, R.K. et al. (1994) Nature 367, 94-97; Swaminathan, S. et al. (1992) Nature 359, 801-806] within two distinct domains. Differences between the proteins are located primarily in highly variable regions comprised of several surface loops, such as the disulfide-bonded loop which is absent from TSST-1 and at the amino terminus.

The X-ray crystal structures of SEB and TSST-1 complexed with HLA DR1 are known [Kim, J. et al. (1994) Science 266, 1870-1874 ; Jardetzky, T.S. et al. (1994) Nature 368, 711-718] and this data was useful to fully explain our results concerning attenuation of the superantigens by site-specific mutagenesis. The region of HLA DR1 that contacts SEB consists exclusively of α subunit surfaces. The main regions of SEB involved are two conserved sites: a polar pocket derived from three β strands of the β barrel domain and a highly solvent-exposed hydrophobic reverse turn. The polar binding pocket of SEB contains a glutamate and two tyrosines that accommodate Lys39 of the α subunit of HLA DR1, while the hydrophobic region consists of a leucine and flanking residues that make several contacts with the HLA DRα chain. The HLA DR1 binding sites of both TSST-1 and SEB overlap significantly. The hydrophobic binding contacts of other SAg with the HLA DRα chain have been proposed [Ulrich et al. (1995) Nature, Struct. Biol. 2, 554-560] to be similar to those found in SEB and TSST-1. A motif consisting of a leucine in a reverse turn [Ulrich *et al.* (1995), supra] is conserved among bacterial superantigens and may provide the key determinant (hydrophobic or otherwise) for binding HLA-DR. However, TSST-1 does not have a highly charged residue in the polar pocket that interacts with Lys39 of the HLA DRα chain and uses an alternative conformational binding mode that allows TSST-1 to interact with HLA DR1 β-chain residues and the carboxy-terminal region of the antigenic peptide.

Both SEA and SEE bind to the β subunit of DR by means of a single zinc atom [Fraser, J.D. et al. (1992) Proc. Natl. Acad. Sci. USA 89, 5507-5511]. The amino-terminal domain of SEA interfaces with the HLA DRα chain [Ulrich *et al.* (1995), supra], while SEA C-terminal domain residues His187, His225 and Asp227 form a zinc-coordination complex, likely with His-81 from the β chain of an adjoining HLA DR molecule. However, our results have shown that binding of superantigen to the HLA DRβ subunit does not directly stimulate T cells [Ulrich *et al.* (1995), supra] but increases the potential of the bound SEA to interact with the α chain of another HLA DR, thus increasing the biological potency.

### EXAMPLE 2

### Molecular modelling and structural studies of staphylococcal and streptococcal superantigens: A detailed protein structure analysis of SEB and SEA suggested that all bacterial superantigens have a common mechanism for binding MHC class II receptors.

A least-squares superimposition of the unbound molecules of modeled SEA and the crystal structure of SEB, aligned according to their structurally conserved α-helical and β-strand regions, exhibited a global folding pattern which is very similar. Differences between the two structures are calculated to be located primarily in loops of low sequence homologies, with the largest positional deviations occurring between structurally conserved regions of residues 18-20, 30-32, 173-181, 191-194, and the cysteine-loop region (90-111). Only one of these regions in SEB makes significant contact (residue Y94 in particular) with the HLA-DR1 molecule [Jardetzky, T.S. et al. (1994) Nature 368, 711-718].

The binding interface between SEB and HLA-DR1 consists principally of two structurally conserved surfaces located in the N-terminal domain: a polar binding pocket derived from three β-strand elements of the β-barrel domain and a hydrophobic reverse turn. The binding pocket of SEB contains residues E67, Y89 and Y115, and binds K39 of the DRα subunit. For SEA, the binding interface with the DR molecule is modeled to contain a similar binding pocket consisting of residues D70, Y92 and Y108. Mutation of residue Y89 in SEB or Y92 in SEA to alanine (**Fig. 2**) resulted in 100-fold reduction in DR1 binding. The substitution of alanine for Y89 in SEB and Y92 in SEA eliminates the hydrogen bond with K39 and disrupts packing interactions with adjacent protein residues. Modeling of the SEA mutant Y92A predicts an increase in solvent-accessible surface area for Y108 by a factor of two greater than the wild-type structure, allowing the formation of a hydrogen bond to the carboxylate group of D70 and thus disrupting key anchoring and recognition points for HLA-DR1. This effect is expected to be somewhat less in SEB due to the longer side chain at E67. Substitution of SEB Y115 with alanine also resulted in 100-fold reduction of binding. In contrast, the same replacement of Y108 in SEA yielded little to no change in DR1 binding (**Fig. 2a**), suggesting the primary importance of SEA residues Y92 and D70 for stabilizing interactions with K39. The K39 side chain of DRα forms a strong ion-pair interaction with the SEB E67 carboxylate group and hydrogen bonds with the hydroxyl groups of Y89 and Y115. Substitution of SEB E67 by glutamine reduced binding affinity by 100-fold (**Fig. 2**), reflecting the replacement of the strong ionic bond with a weaker hydrogen bond. To optimize ion-pair interactions of the analogous SEA site, the shorter carboxylate side chain of D70 is predicted to shift K39 of DRα, weakening interactions with SEA Y108. The substitution of alanine for SEA Y108 is thus more easily accommodated than the homologous substitution of SEB Y115, without loss in DR1 binding.

Comparisons of the polar pocket with other bacterial superantigens were then made. SEC1-3 and SPE-A have conserved the critical DR1 binding-interface residues (**Fig. 1**), and share with SEB and SEA secondary structural elements of the DR1-binding surfaces. Asparagine in SED (N70) replaces the acidic side chain present in SEA, SEB, SPE-A and SEC1-3. Accordingly, for SED the salt bridge of the polar pocket is likely to be replaced by a hydrogen bond. Overall DR1 affinities for SED and SEA appeared to be equivalent (**Fig. 2b**), indicating that other interactions may compensate for the absence in SED of the ion-pair found in the other superantigens. For the case of TSST-1, mutating DRα residues K39 to serine or M36 to isoleucine has been shown to greatly reduce binding [Panina-Bordignon et al. (1992) J. Exp. Med. 176: 1779-1784]. Although primarily hydrophobic, the critical TSST-1 structural elements are conserved with the SEA and SEB polar binding pocket. SEB residues Y89 and Y115 are homologous to T69 and I85 in TSST-1, respectively, and SEB E67 is replaced by 146. These TSST-1 residues are positioned in a conserved ß-barrel domain found in both SEB and SEA. However, the TSST-1 site lacks polarity equivalent to SEB/SEA, and hydrogen bonding with the hydroxyl of TSST-1 residue T69 would require that DRα K39 extend 5 Å into the pocket. TSST-1 binding utilizes an alternative strategy [Kim et al. (1994) Science 266: 1870-1874] consisting of hydrophobic contacts centered around residue I46, and potential ionic or hydrogen bonds bridging DRα residues E71 and K67 to R34 and D27, respectively, of TSST-1.

The hydrophobic region of the binding interface between SEB and the HLA-DR1 molecule consists of SEB residues 44-47, located in a large reverse turn connecting 9-stands 1 and 2 of SEB. These residues appear to make strong electrostatic interactions with DRα through their backbone atoms. The mutation of L45 to an arginine reduced overall HLA-DR1 binding greater than 100-fold (**Fig. 2b**), attributable to the less energetically favorable insertion of a highly charged residue into a hydrophobic depression on the DR1 molecule. The modeled DR1-SEA complex presents similar interactions with the SEA backbone atoms, with the exception of a glutamine (Q49) replacing SEB Y46. Mutation of L48 to glycine in SEA (homologous to L45 of SEB) has been reported to decrease T-cell responses. SEB L45 and the comparable L30 of TSST-1 are the most extensively buried residues in the DR1 interface. The leucine is conserved among the bacterial superantigens (**Fig. 3**) and may provide the necessary hydrophobic structural element for surface complementarity with DR1, consistent with the mutagenesis data for SEB and SEA.

The inventor has performed similar structure and function studies with TSST-1, SEC1 and SPE-A.

### EXAMPLE 13

### Design of altered SpeA toxin vaccine, SpeA42

Streptococcal pyrogenic exotoxin A (SpeA) is produced by group A *Streptococcus pyogenes* and is associated with outbreaks of streptococcal toxic shock syndrome. SpeA is also a virulence factor for invasive infections. The M1inv+ subclone of M1 GAS that spread globally in the late 1980s and early 1990s harbors the phage T14 that encodes the superantigen streptococcal pyrogenic exotoxin A or SpeA (Infect. Immun. 66:5592 (1998). A typical bacterial superantigen, the 25,700 Mᵣ secreted SpeA polypeptide aids in immune escape by targeting the primary step in immune recognition. The cellular receptors are human major histocompatibility complex (MHC) class II molecules, primarily HLA-DR, and T-cell antigen receptors (TCRs). The normal antigen-specific signal transduction of T cells is disengaged by the superantigen, which acts as a wedge to prevent contacts of MHC-bound, antigenic peptides with specific combining site elements of the TCR. The magnitude of the T-cell response is significantly greater than antigen-specific activation and results in pathological levels of proinflammatory cytokines such as tumor necrosis factor alpha (TNF-α) and interferon-γ.

Clinical isolates of *Streptococcus pyogenes* harboring the SpeA gene were identified by PCR amplification of a sequence-specific fragment from bacterial DNA. Specific restriction enzyme motifs for cloning were introduced into the amplified DNA fragment by using the following oligonucleotide primers: 5' CTCG CAA GAG GTA CAT ATG CAA CAA GAC 3' (SEQ ID NO:17), sense primer to introduce a unique NdeI site; 5' GCA GTA GGT AAG CTT GCC AAA AGC 3' (SEQ ID NO:18), antisense primer to introduce a unique Hind III site. The amplified DNA fragment was ligated into the EcoRI site of a PCR-cloning plasmid (Perfectly Blunt, Invitrogen) and the resulting plasmid was used to transform *E. coli* host strain DH5α The HindIII/EcoRI DNA fragment containing the full-length SpeA gene minus the signal peptide was cloned into pET24 vector for expression in *E. coli* host strain BL21. Although the mutant proteins can be produced with the leader peptide sequence present, deletion of the leader peptide appeared to produce a higher yield of protein. Proteins were purified from *E. coli* inclusions and purified by cationic/anionic-exchange chromatography using standard methods (Coffman, J.D. et al., 2001. Prot. Express. Purif. in press). The method of purification was not critical to the performance of the vaccine. Lipopolysaccharide contaminants, resulting from expression in a Gram-negative bacterium, were readily removed (as determined by limulus assay) using a variety of standard methods. Two different mutants of SpeA were designed and produced based on the principle of mutating key amino acid residues involved with binding to MHC class II receptors. The first SpeA construct consists of a single mutation at residue leucine 42, while the second construct consists of a fusion between the SpeA mutant of leucine 42 and a mutant SpeB protein.

The binding interface between SpeA and HLA-DR is predicted to consist of contacts located in the N-terminal domain that are conserved with other bacterial superantigens. Leucine 42 of SpeA is predicted to protrude from a reverse turn on the surface of SpeA and form a major hydrophobic contact with the HLA-DR receptor molecule. Mutation of the single residue leucine 42 in SpeA to the charged amino acid side chain of arginine is predicted to disrupt this major contact with the receptor molecule, resulting in a significant reduction in DR1 binding. This mutant molecule should therefore have lost the toxin attributes of the wild-type molecule. Mutations of SpeA at amino acid position 42 (e.g. L42R or L42A) resulted in greatly diminished interactions with cell surface HLA-DR, as measured by laser fluorescence-activated flow cytometry and FITC-labeled rabbit anti-SpeA antibody (affinity purified). Human T-cell proliferation in response to these mutants was next assessed by [³H]thymidine incorporation, using a 12 h pulse with label and harvesting cells after 60 h of culture. Mutations of SpeA at amino acid position 42 (L42R or L42A) resulted in greatly diminished activation of human lymphocytes (Figure 10). Although alanine or arginine substitutions of L42 were indistinguishable by MHC class II binding, arginine substitution (L42R) resulted in the greatest attenuation of T-cell responses.

### EXAMPLE 14

### Design of the SpeA-SpeB fusion antigen/vaccine

The vast majority of *Streptococcus pyogenes* isolates express an extracellular cysteine protease historically termed streptococcal pyrogenic exotoxin B (SpeB). The protease is an important colonization and pathogenicity factor (Kuo, C.-F. et al., 1998, Infect. Immun. 66: 3931-35). However, co-purification of contaminant streptococcal proteins with SpeB led to the erroneous conclusion that the protease was a superantigen. Several potential host substrates are known. For example, the purified SpeB cleaves interleukin 1 precursor protein to produce active interleukin 1 and also cleaves the extracellular matrix proteins fibronectin and vitronectin (Kapur, V. et al. 1993, Microb. Path. 15: 327-346; Kapur, V., et al.,1993, Proc. Natl. Acad. Sci. U.S.A. 90: 7676-80). The ubiquitous expression of SpeB by *S. pyogenes* and the conserved nature of the antigenic determinants recognized by antibodies are noteworthy features. Although multiple alleles exist, polyclonal antisera generated against one SpeB allelic product reacts with SpeB from all *S. pyogenes* M1 serotypes examined (Proc. Natl. Acad. Sci. U.S.A. 90: 7676-80). Based on analysis of the catalytic site structure from crystallographic data (T. F. Kagawa, et al., 2000, Proc. Natl. Acad. Sci. USA 97:2235-2240) mutation of active-site residues, for example cysteine at position 47 or histidine at position 340, inactivates proteolytic activity (T. F. Kagawa *et al*. *supra;* Gubba, S.et al., 2000, Infect Immun. 68:3716-9). A mutant, catalytically inactive SpeB (SpeB C47S) was used as a fusion partner with mutant SpeA (SpeA L42R).

The wild-type SpeB zymogen, cloned from a clinical isolate of GAS, was truncated by PCR cloning to produce the mature protein minus the noncatalytic prosegment domain. An additional construct was designed to incorporate the prosegment in the final SpeA-B fusion. Because of solubility problems, only the SpeB minus the prosegment was used for support data. A mutant, catalytically inactive SpeB was constructed by site specific mutagenesis of the DNA coding sequence, altering cysteine at amino acid position 47 to serine. This conservative change maintains the approximate dimensions of the active-site side chain but prevents proteolytic activity. SpeB C47S) was used as a fusion partner with mutant SpeA (SpeA L42R). A pfu DNA polymerase was used for all PCR reactions to lessen the likelihood of introducing spurious mutations common with lower fidelity polymerases, e.g. *taq*. For cloning, the SpeA (L43R) gene was used as a PCR template and primers 1 SEQ ID NO:19) and 2 (SEQ ID NO:20) were used to prepare a double-stranded sequence overlapping with SpeB(C47S). A separate PCR reaction using primers 3 (SEQ ID NO:21) and 4 (SEQ ID NO:22) and SpeB (C47S) gene insert was performed to generate a double-stranded DNA fragment overlapping with SpeA (L42R). The PCR fragments were purified by agarose gel electrophoresis and mixed together for a final PCR reaction using primers 1 and 4, to create the full-length gene fusion of SpeA (L42R)-SpeB (C47S). This full-length fragment was blunt-end cloned into the vector pT7Blue (Novagen) and sequence confirmed (SEQ ID NO: 23). The SpeA (L42R)-SpeB (C47S) fusion gene was then subcloned into pET24b(+) for expression in *E. coli* BL21 host strains. The SpeB clone, prosegment plus mature polypeptide is presented in SEQ ID NO:24. The mature SpeB polypeptide used for the SpeA-SpeB fusion is identified in SEQ ID NO:25. The SpeA (L42R) used for the SpeA-SpeB fusion is identified in SEQ ID NO:26. The amino acid sequence of the SpeA-SpeB fusion is identified in SEQ ID NO:27. SEQ ID NO:28-31 identify primers used in the preparation of the SpeA-SpeB fusion, where SpeB prosegment and mature protein were fused with SpeA.

The potential advantages to this fusion construct above the non-fused SpeA mutant are: better activation of immune responses, immune protection against a second virulence factor, cost savings and simplification of product production. The predicted 54 kDa protein was detected by polyacrylamide gel electrophoresis and Coomassie Blue staining. Antibodies specific for either SpeA or SpeB both detected the SpeA L42R-SpeB C47S fusion protein by Western blot analysis.

### EXAMPLE 15

Mouse antibody response to SpeA L42R and SpeA-B fusion constructs. Because only minor changes have been introduced into the final protein product, maximum antigenicity is maintained. Immune recognition of the SpeA mutants was next examined in an LPS-dependent, murine toxicity model previously described (Stiles, B.G., 1993, Infect. Immun. 61:5333). BALB/c mice (female, 20 grams average weight; NCI) were vaccinated three times with 10 µg of each construct, allowing two weeks between injections. Sera from each experimental group (n=5) were pooled for measurement of specific antibodies. Data shown in Figure 11 are antigen-specific antibodies (ELISA units) present in a 1:100,000 dilution of pooled sera from mice vaccinated with SpeA L42R, SpeA-B fusion or adjuvant only. BALB/c mice were vaccinated three times with 10 µg of each construct, allowing two weeks between injections. Vaccination with either SpeA L42R or the SpeA-B fusion produced high antibody titers. As anticipated, antibodies from SpeA L42R vaccination only recognized SpeA, whereas, antibodies from the SpeA-B fusion vaccinated mice recognized both SpeA and SpeB. Although these data confirmed the potent immunogenicity of the SpeA constructs, the inbred mouse was an inadequate model to demonstrate protective immunity. Within reasonable physiological limits, wild-type SpeA was not lethal for several inbred mouse strains examined. Therefore, a transgenic model was used consisting of mice (H-2^{b} background) expressing human CD4 and HLA-DQ8 (Taneja, V., and C. S. David. 1999, Immunol Rev 169:67; Nabozny, G. H., et al., 1996, J Exp Med 183:27). With these transgenic mice SpeA wild-type was lethal at relatively low concentrations, and the SpeA mutant constructs were also highly immunogenic. HLA-DQ is structurally very similar to HLA-DR, although crystallographic data were not available for the previous molecular modeling studies used for designing the mutant superantigen. Proliferative responses were examined using mononuclear cells isolated from spleens of transgenic mice expressing HLA-DR3, HLA-DQ8 or HLA-DR2β/IEα, or non-transgenic BALB/c mice and human peripheral blood (Figure 12). These in vitro responses of the HLA-DQ+ mice were very similar to results obtained with human mononuclear cells. BALB/c or hemi-transgenic mice in which the mouse IEα was paired with the human HLA-DR2β subunit required greater amounts of wild-type SpeA to produce a level of proliferation equivalent to the HLA-DQ8 transgenes. Non-vaccinated HLA-DQ8 mice were very sensitive to SpeA challenges, whereas, vaccination with SpeA L42R or the SpeA-B construct fully protected HLA-DQ8 transgenic mice from challenge with the same amount of wild-type SpeA.

**Table 13: SpeA Vaccination and Immune Protection: HLA-DQ8/human CD4 Transgenic Mice**

| | |
|---|---|
| Vaccination¹ | Challenge Survival² |
| | |
| SpeA L42R | 100% |
| | |
| SpeA-B fusion | 100% |
| | |
| Adjuvant only control 3 doses | 0% |
| | |

| | |
|---|---|
| ¹Vaccinations with 10 µg L30R, L30A in adjuvant (Alhydrogel) or adjuvant only per mouse 0,2 and 4 weeks (3 dose), ²SpeA wild-type i.p. dose 5 LD₅₀ per mouse 2 weeks after last vaccination. Percent survivors by 72 hours. 5 mice per group SpeA L42R and adjuvant only control; 4 mice for SpeA-B fusion vaccination. Experiments involving SpeA L42R were performed twice (n=5 mice per group) with identical results; experiments involving SpeA-B fusion vaccine was performed once. | |

### SEQUENCE LISTING

(1) GENERAL INFORMATION:
   (i) APPLICANT: Robert G. Ulrich
   (ii) TITLE OF INVENTION: Bacterial Superantigen Vaccines
   (iii) NUMBER OF SEQUENCES:31
   (iv) CORRESPONDENCE ADDRESS:
      (A) ADDRESSEE: Elizabeth Arwine
      (B) STREET: US Army MRMC -504 Scott Street MCMR-JA (Elizabeth Arwine-Patent Atty)
      (C) CITY: FORT DETRICK
      (D) STATE: MARYLAND
      (E) COUNTRY: USA
      (F) ZIP: 21702-5012
   (v) COMPUTER READABLE FORM:
      (A) MEDIUM TYPE: Floppy disk
      (B) COMPUTER: Apple Macintosh
      (C) OPERATING SYSTEM: Macintosh 7.5
      (D) SOFTWARE: Microsoft Word 6.0
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/882,431
      (B) FILING DATE: June 25, 1997
   (viii) ATTORNEY/AGENT INFORMATION:
      (A) NAME: Sana A. Pratt
      (B) REGISTRATION NUMBER: 39,441
      (C) REFERENCE/DOCKET NUMBER:
   (ix) TELECOMMUNICATION INFORMATION
      (A) TELEPHONE: (301) 619-2065
      (B) TELEFAX: (301) 619-7714
(2) INFORMATION FOR SEQUENCE ID NO:1:
   (i) SEQUENCE CHARACTERISTICS:
      (A) LENGTH: 830
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:1:
   ATGAAAAAAA CAGCATTTAC ATTACTTTTA TTCATTGCCC     40
   TAACGTTGAC AACAAGTCCA CTTGTAAATG GTAGCGAGAA     80
   AAGCGAAGAA ATAAATGAAA AAGATTTGCG AAAAAAGTCT    120
   GAATTGCAGG GAACAGCTTT AGGCAATCTT AAACAAATCT    160
   ATTATTACAA TGAAAAAGCT AAAACTGAAA ATAAAGAGAG    200
   TCACGATCAA TTTCGACAGC ATACTATATT GTTTAAAGGC    240
   TTTTTTACAG ATCATTCGTG GTATAACGAT TTATTAGTAC    280
   GTTTTGATTC AAAGGATATT GTTGATAAAT ATAAAGGGAA    320
   AAAAGTAGAC TTGTATGGTG CTTATGCTGG TTATCAATGT    360
   GCGGGTGGTA CACCAAACAA AACAGCTTGT ATGTATGGTG    400
   GTGTAACGTT ACATGATAAT AATCGATTGA CCGAAGAGAA    440
   AAAAGTGCCG ATCAATTTAT GGCTAGACGG TAAACAAAAT    480
   ACAGTACCTT TGGAAACGGT TAAAACGAAT AAGAAAAATG    520
   TAACTGTTCA GGAGTTGGAT CTTCAAGCAA GACGTTATTT    560
   ACAGGAAAAA TATAATTTAT ATAACTCTGA TGTTTTTGAT    600
   GGGAAGGTTC AGAGGGGATT AATCGTGTTT CATACTTCTA    640
   CAGAACCTTC GGTTAATTAC GATTTATTTG GTGCTCAAGG    680
   ACAGTATTCA AATACACTAT TAAGAATATA TAGAGATAAT    720
   AAAACGATTA ACTCTGAAAA CATGCATATT GATATATATT    760
   TATATACAAG TTAAACATGG TAGTTTTGAC CAACGTAATG    800
   TTCAGATTAT TATGAACCGA GAATAATCTA               830
(3) INFORMATION FOR SEQUENCE ID NO:2:
   (i) SEQUENCE CHARACTERISTICS:
      (A) LENGTH: 257
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:2:
   Met Lys Lys Thr Ala Phe Thr Leu Leu Leu
   1 5 10
   Phe Ile Ala Leu Thr Leu Thr Thr Ser Pro
   15 20
   Leu Val Asn Gly Ser Glu Lys Ser Glu Glu
   25 30
   Ile Asn Glu Lys Asp Leu Arg Lys Lys Ser
   35 40
   Glu Leu Gln Gly Thr Ala Leu Gly Asn Leu
   45 50
   Lys Gln Ile Tyr Tyr Tyr Asn Glu Lys Ala
   55 60
   Lys Thr Glu Asn Lys Glu Ser His Asp Gln
   65 70
   Phe Arg Gln His Thr Ile Leu Phe Lys Gly
   75 80
   Phe Phe Thr Asp His Ser Trp Tyr Asn Asp
   85 90
   Leu Leu Val Arg Phe Asp Ser Lys Asp Ile
   95 100
   Val Asp Lys Tyr Lys Gly Lys Lys Val Asp
   105 110
   Leu Tyr Gly Ala Tyr Ala Gly Tyr Gln Cys
   115 120
   Ala Gly Gly Thr Pro Asn Lys Thr Ala Cys
   125 130
   Met Tyr Gly Gly Val Thr Leu His Asp Asn
   135 140
   Asn Arg Leu Thr Glu Glu Lys Lys Val Pro
   145 150
   Ile Asn Leu Trp Leu Asp Gly Lys Gln Asn
   155 160
   Thr Val Pro Leu Glu Thr Val Lys Thr Asn
   165 170
   Lys Lys Asn Val Thr Val Gln Glu Leu Asp
   175 180
   Leu Gln Ala Arg Arg Tyr Leu Gln Glu Lys
   185 190
   Tyr Asn Leu Tyr Asn Ser Asp Val Phe Asp
   195 200
   Gly Lys Val Gln Arg Gly Leu Ile Val Phe
   205 210
   His Thr Ser Thr Glu Pro Ser Val Asn Tyr
   215 220
   Asp Leu Phe Gly Ala Gln Gly Gln Tyr Ser
   225 230
   Asn Thr Leu leu Arg Ile Tyr Arg Asp Asn
   235 240
   Lys Thr Ile Asn Ser Glu Asn Met His Ile
   245 250
   Asp Ile Tyr Leu Tyr Thr Ser
   255
(4) INFORMATION FOR SEQUENCE ID NO:3:
   (i) SEQUENCE CHARACTERISTICS:
      (A) LENGTH: 757
      (B) TYPE: Nucleic Acid
      (C) STRANDEDNESS: Unknown
      (D) TOPOLOGY: Unknown
   (ii) Molecule type: DNA (xi) SEQUENCE DESCRIPTION: SEQ ID NO:3:
(5) INFORMATION FOR SEQUENCE ID NO:4:
   (i) SEQUENCE CHARACTERISTICS:
      (A) LENGTH:233
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:4:
   Met Glu Lys Ser Glu Glu Ile Asn Glu Lys
                   5                   10
   Asp Leu Arg Lys Lys Ser Glu Leu Gln Gly
                   15                  20
   Thr Ala Leu Gly Asn Leu Lys Gln Ile Tyr
                   25                  30
   Tyr Tyr Asn Glu Lys Ala Lys Thr Glu Asn
                   35                  40
   Lys Glu Ser His Asp Gln Phe Arg Gln His
                   45                  50
   Thr Ile Leu Phe Lys Gly Phe Phe Thr Asp
   55 60
   His Ser Trp Tyr Asn Asp Leu Leu Val Arg
   65 70
   Phe Asp Ser Lys Asp Ile Val Asp Lys Tyr
   75 80
   Lys Gly Lys Lys Val Asp Leu Tyr Gly Ala
   85 90
   Tyr Ala Gly Tyr Gln Cys Ala Gly Gly Thr
   95 100
   Pro Asn Lys Thr Ala Cys Met Tyr Gly Gly
   105 110
   Val Thr Leu His Asp Asn Asn Arg Leu Thr
   115 120
   Glu Glu Lys Lys Val Pro Ile Asn Leu Trp
   125 130
   Leu Asp Gly Lys Gln Asn Thr Val Pro Leu
   135 140
   Glu Thr Val Lys Thr Asn Lys Lys Asn Val
   145 150
   Thr Val Gln Glu Leu Asp Leu Gln Ala Arg
   155 160
   Arg Tyr Leu Gln Glu Lys Tyr Asn Leu Tyr
   165 170
   Asn Ser Asp Val Phe Asp Gly Lys Val Gln
   175 180
   Arg Gly Leu Ile Val Phe His Thr Ser Thr
   185 190
   Glu Pro Ser Val Asn Tyr Asp Leu Phe Gly
   195 200
   Ala Gln Gly Gln Tyr Ser Asn Thr Leu Leu
   205 210
   Arg Ile Tyr Arg Asp Asn Lys Thr Ile Asn
   215 220
   Ser Glu Asn Met His Ile Asp Ile Tyr Leu
   225 230
   Tyr Thr Ser
(6) INFORMATION FOR SEQUENCE ID NO:5:
   (i) SEQUENCE CHARACTERISTICS:
      (A) LENGTH: 1712
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:5:
   GAACTAGGTA GAAAAATAAT TATGAGAAAA CACTATGTTG 40
   TTAAAGATGT TTTCGTATAT AAGTTTAGGT GATGTATAGT 80
   TACTTAATTT TAAAAGCATA ACTTAATTAA TATAAATAAC 120
   ATGAGATTAT TAAATATAAT TAAGTTTCTT TTAATGTTTT 160
   TTTAATTGAA TATTTAAGAT TATAACATAT ATTTAAAGTG 200
   TATCTAGATA CTTTTTGGGA ATGTTGGATA AAGGAGATAA 240
   AAAATGTATA AGAGATTATT TATTTCACAT GTAATTTTGA 280
   TATTCGCACT GATATTAGTT ATTTCTACAC CCAACGTTTT 320
   AGCAGAGAGT CAACCAGATC CTAAACCAGA TGAGTTGCAC 360
   AAATCGAGTA AATTCACTGG TTTGATGGAA GATATGAAAG 400
   TTTTGTATGA TGATAATCAT GTATCAGCAA TAAACGTTAA 440
   ATCTATAGAT CAATTTCTAT ACTTTGACTT AATATATTCT 480
   ATTAAGGACA CTAAGTTAGG GGATTATGAT AATGTTCGAG 520
   TCGAATTTAA AAACAAAGAT TTAGCTGATA AATACAAAGA 560
   TAAATACGTA GATGTGTTTG GAGCTAATTA TTATTATCAA 600
   TGTTATTTTT CTAAAAAAAC GAATGATATT AATTCGCATC 640
   AAACTGACAA ACGAAAAACT TGTATGTATG GTGGTGTAAC 680
   TGAGCATAAT GGAAACCAAT TAGATAAATA TAGAAGTATT 720
   ACTGTTCGGG TATTTGAAGA TGGTAAAAAT TTATTATCTT    760
   TTGACGTACA AACTAATAAG AAAAAGGTGA CTGCTCAAGA    800
   ATTAGATTAC CTAACTCGTC ACTATTTGGT GAAAAATAAA    840
   AAACTCTATG AATTTAACAA CTCGCCTTAT GAAACGGGAT    880
   ATATTAAATT TATAGAAAAT GAGAATAGCT TTTGGTATGA    920
   CATGATGCCT GCACCAGGAG ATAAATTTGC CCAATCTAAA    960
   TATTTAATGA TGTACAATGA CAATAAAATG GTTGATTCTA   1000
   AAGATGTGAA GATTGAAGTT TATCTTACGA CAAAGAAAAA   1040
   GTGAAATTAT ATTTTAGAAA AGTAAATATG AAGAGTTAGT   1080
   AATTAAGGCA GGCACTTATA GAGTACCTGC CTTTTCTAAT   1120
   ATTATTTAGT TATAGTTATT TTTGTTATAT CTCTCTGATT   1160
   TAGCATTAAC CCCTTGTTGC CATTATAGTT TTCACCAACT   1200
   TTAGCTGAAA TTGGGGGATC ATTTTTATCT TTACTATGGA   1240
   TAGTTACTGT GTCGCCGTTT TTAACGATTT GTTTCTCTTT   1280
   TAATTTGTCA GTTAATTTTT TCCATGCATC ATTTGCGTCA   1320
   AACCTATTTC CATTTGGATT TATTCTTGAC AAATCAATTC   1360
   TTTTAACACT ATCGGTATTA ATCGGCTTGT TATTAAAATT   1400
   ACTAAGTTCA TCTAAATCAG CTGTACCCGT AATACTACTT   1440
   TCGCCACCAT TATTTAAATT GTACGTAACA CCAACTGTCT   1480
   CATTTGCTGT TTTATCGATA ATATTTGCTT CTTTCAAAGC   1520
   ATCTCTTACA TTTTTCCATA AGTCTCTATC TGTTATTTCA   1560
   GAAGCCTTTG CAACGTTATT AATACCATTA TAATTTGAAG   1600
   AAGAATGAAA ACCTGAACCT ACTGTTGTTA AAACTAAAGC   1640
   ACTTGCTATC AATGTTCTTG TTAATAGTTT TTTATTCATT   1680
   TTATTTTCTC CTATAACTTA TTTGCAATCG AT           1712
(7) INFORMATION FOR SEQUENCE ID NO: 6:
   (i) SEQUENCE CHARACTERISTICS:
      (A) LENGTH: 266
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:6:
   Met Tyr Lys Arg Leu Phe Ile Ser His Val
                    5                  10
   Ile Leu Ile Phe Ala Leu Ile Leu Val Ile
                   15                  20
   Ser Thr Pro Asn Val Leu Ala Glu Ser Gln
                   25                  30
   Pro Asp Pro Lys Pro Asp Glu Leu His Lys
                   35                  40
   Ser Ser Lys Phe Thr Gly Leu Met Glu Asp
                   45                  50
   Met Lys Val Leu Tyr Asp Asp Asn His Val
                   55                  60
   Ser Ala Ile Asn Val Lys Ser Ile Asp Gln
                   65                  70
   Phe Leu Tyr Phe Asp Leu Ile Tyr Ser Ile
                   75                  80
   Lys Asp Thr Lys Leu Gly Asp Tyr Asp Asn
                   85                  90
   Val Arg Val Glu Phe Lys Asn Lys Asp Leu
                   95                 100
   Ala Asp Lys Tyr Lys Asp Lys Tyr Val Asp
                  105                 110
   Val Phe Gly Ala Asn Tyr Tyr Tyr Gln Cys
                  115                 120
   Tyr Phe Ser Lys Lys Thr Asn Asp Ile Asn
                  125                 130
   Ser His Gln Thr Asp Lys Arg Lys Thr Cys
                  135                 140
   Met Tyr Gly Gly Val Thr Glu His Asn Gly
                  145                 150
   Asn Gln Leu Asp Lys Tyr Arg Ser Ile Thr
   155 160
   Val Arg Val Phe Glu Asp Gly Lys Asn Leu
   165 170
   Leu Ser Phe Asp Val Gln Thr Asn Lys Lys
   175 180
   Lys Val Thr Ala Gln Glu Leu Asp Tyr Leu
   185 190
   Thr Arg His Tyr Leu Val Lys Asn Lys Lys
   195 200
   Leu Tyr Glu Phe Asn Asn Ser Pro Tyr Glu
   205 210
   Thr Gly Tyr Ile Lys Phe Ile Glu Asn Glu
   215 220
   Asn Ser Phe Trp Tyr Asp Met Met Pro Ala
   225 230
   Pro Gly Asp Lys Phe Ala Gln Ser Lys Tyr
   235 240
   Leu Met Met Tyr Asn Asp Asn Lys Met Val
   245 250
   Asp Ser Lys Asp Val Lys Ile Glu Val Tyr
   255 260
   Leu Thr Thr Lys Lys Lys
   265
(8) INFORMATION FOR SEQUENCE ID NO:7:
   (i) SEQUENCE CHARACTERISTICS:
      (A) LENGTH: 1712
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:7:
   GAACTAGGTA GAAAAATAAT TATGAGAAAA CACTATGTTG 40
   TTAAAGATGT TTTCGTATAT AAGTTTAGGT GATGTATAGT 80
   TACTTAATTT TAAAAGCATA ACTTAATTAA TATAAATAAC 120
   ATGAGATTAT TAAATATAAT TAAGTTTCTT TTAATGTTTT 160
   TTTAATTGAA TATTTAAGAT TATAACATAT ATTTAAAGTG 200
   TATCTAGATA CTTTTTGGGA ATGTTGGATA AAGGAGATAA 240
   AAAATGTATA AGAGATTATT TATTTCACAT GTAATTTTGA 280
   TATTCGCACT GATATTAGTT ATTTCTACAC CCAACGTTTT 320
   AGCAGAGAGT CAACCAGATC CTAAACCAGA TGAGTTGCAC 360
   AAATCGAGTA AATTCACTGG TTTGATGGAA AATATGAAAG 400
   TTTTGTATGA TGATAATCAT GTATCAGCAA TAAACGTTAA 440
   ATCTATAGAT CAATTTCGAT ACTTTGACTT AATATATTCT 480
   ATTAAGGACA CTAAGTTAGG GAATTATGAT AATGTTCGAG 520
   TCGAATTTAA AAACAAAGAT TTAGCTGATA AATACAAAGA 560
   TAAATACGTA GATGTGTTTG GAGCTAATGC TTATTATCAA 600
   TGTGCTTTTT CTAAAAAAAC GAATGATATT AATTCGCATC 640
   AAACTGACAA ACGAAAAACT TGTATGTATG GTGGTGTAAC 680
   TGAGCATAAT GGAAACCAAT TAGATAAATA TAGAAGTATT 720
   ACTGTTCGGG TATTTGAAGA TGGTAAAAAT TTATTATCTT 760
   TTGACGTACA AACTAATAAG AAAAAGGTGA CTGCTCAAGA 800
   ATTAGATTAC CTAACTCGTC ACTATTTGGT GAAAAATAAA 840
   AAACTCTATG AATTTAACAA CTCGCCTTAT GAAACGGGAT 880
   ATATTAAATT TATAGAAAAT GAGAATAGCT TTTGGTATGA 920
   CATGATGCCT GCACCAGGAG ATAAATTTGA CCAATCTAAA 960
   TATTTAATGA TGTACAATGA CAATAAAATG GTTGATTCTA 1000
   AAGATGTGAA GATTGAAGTT TATCTTACGA CAAAGAAAAA 1040
   GTGAAATTAT ATTTTAGAAA AGTAAATATG AAGAGTTAGT 1080
   AATTAAGGCA GGCACTTATA GAGTACCTGC CTTTTCTAAT 1120
   ATTATTTAGT TATAGTTATT TTTGTTATAT CTCTCTGATT 1160
   TAGCATTAAC CCCTTGTTGC CATTATAGTT TTCACCAACT   1200
   TTAGCTGAAA TTGGGGGATC ATTTTTATCT TTACTATGGA   1240
   TAGTTACTGT GTCGCCGTTT TTAACGATTT GTTTCTCTTT   1280
   TAATTTGTCA GTTAATTTTT TCCATGCATC ATTTGCGTCA   1320
   AACCTATTTC CATTTGGATT TATTCTTGAC AAATCAATTC   1360
   TTTTAACACT ATCGGTATTA ATCGGCTTGT TATTAAAATT   1400
   ACTAAGTTCA TCTAAATCAG CTGTACCCGT AATACTACTT   1440
   TCGCCACCAT TATTTAAATT GTACGTAACA CCAACTGTCT   1480
   CATTTGCTGT TTTATCGATA ATATTTGCTT CTTTCAAAGC   1520
   ATCTCTTACA TTTTTCCATA AGTCTCTATC TGTTATTTCA   1560
   GAAGCCTTTG CAACGTTATT AATACCATTA TAATTTGAAG   1600
   AAGAATGAAA ACCTGAACCT ACTGTTGTTA AAACTAAAGC   1640
   ACTTGCTATC AATGTTCTTG TTAATAGTTT TTTATTCATT   1680
   TTATTTTCTC CTATAACTTA TTTGCAATCG AT           1712
(9) INFORMATION FOR SEQUENCE ID NO:8:
   (i) SEQUENCE CHARACTERISTICS:
      (A) LENGTH: 266
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:8:
   Met Tyr Lys Arg Leu Phe Ile Ser His Val
                   5                   10
   Ile Leu Ile Phe Ala Leu Ile Leu Val Ile
                   15                  20
   Ser Thr Pro Asn Val Leu Ala Glu Ser Gln
                   25                  30
   Pro Asp Pro Lys Pro Asp Glu Leu His Lys
   35 40
   Ser Ser Lys Phe Thr Gly Leu Met Glu Asn
   45 50
   Met Lys Val Leu Tyr Asp Asp Asn His Val
   55 60
   Ser Ala Ile Asn Val Lys Ser Ile Asp Gln
   65 70
   Phe Arg Tyr Phe Asp Leu Ile Tyr Ser Ile
   75 80
   Lys Asp Thr Lys Leu Gly Asn Tyr Asp Asn
   85 90
   Val Arg Val Glu Phe Lys Asn Lys Asp Leu
   95 100
   Ala Asp Lys Tyr Lys Asp Lys Tyr Val Asp
   105 110
   Val Phe Gly Ala Asn Ala Tyr Tyr Gln Cys
   115 120
   Ala Phe Ser Lys Lys Thr Asn Asp Ile Asn
   125 130
   Ser His Gln Thr Asp Lys Arg Lys Thr Cys
   135 140
   Met Tyr Gly Gly Val Thr Glu His Asn Gly
   145 150
   Asn Gln Leu Asp Lys Tyr Arg Ser Ile Thr
   155 160
   Val Arg Val Phe Glu Asp Gly Lys Asn Leu
   165 170
   Leu Ser Phe Asp Val Gln Tyr Asn Lys Lys
   175 180
   Lys Val Thr Ala Gln Glu Leu Asp Tyr Leu
   185 190
   Thr Arg His Tyr Leu Val Lys Asn Lys Lys
   195 200
   Leu Tyr Glu Phe Asn Asn Ser Pro Tyr Glu
   205 210
   Thr Gly Tyr Ile Lys Phe Ile Glu Asn Glu
                   215                     220
   Asn Ser Phe Trp Tyr Asp Met Met Pro Ala
                   225                     230
   Pro Gly Asp Lys Phe Asp Gln Ser Lys Tyr
                   235                     240
   Leu Met Met Tyr Asn Asp Asn Lys Met Val
                   245                     250
   Asp Ser Lys Asp Val Lys Ile Glu Val Tyr
                   255                     260
   Leu Thr Thr Lys Lys Lys
                   265
(10) INFORMATION FOR SEQUENCE ID NO:9:
   (i) SEQUENCE CHARACTERISTICS:
      (A) LENGTH: 1388
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:9:
(11) INFORMATION FOR SEQUENCE ID NO:10:
   (i) SEQUENCE CHARACTERISTICS:
      (A) LENGTH: 239
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:10:
   Met Ser Gln Pro Asp Pro Lys Pro Asp Glu
   5 10
   Leu His Lys Ser Ser Lys Phe Thr Gly Leu
   15 20
   Met Glu Asn Met Lys Val Leu Tyr Asp Asp
   25 30
   Asn His Val Ser Ala Ile Asn Val Lys Ser
   35 40
   Ile Asp Gln Phe Arg Tyr Phe Asp Leu Ile
   45 50
   Tyr Ser Ile Lys Asp Thr Lys Leu Gly Asn
   55 60
   Tyr Asp Asn Val Arg Val Glu Phe Lys Asn
   65 70
   Lys Asp Leu Ala Asp Lys Tyr Lys Asp Lys
   75 80
   Tyr Val Asp Val Phe Gly Ala Asn Ala Tyr
   85 90
   Tyr Gln Cys Ala Phe Ser Lys Lys Thr Asn
   95 100
   Asp Ile Asn Ser His Gln Thr Asp Lys Arg
   105 110
   Lys Thr Cys Met Tyr Gly Gly Val Thr Glu
   115 120
   His Asn Gly Asn Gln Leu Asp Lys Tyr Arg
   125 130
   Ser Ile Thr Val Arg Val Phe Glu Asp Gly
   135 140
   Lys Asn Leu Leu Ser Phe Asp Val Gln Thr
   145 150
   Asn Lys Lys Lys Val Thr Ala Gln Glu Leu
   155 160
   Asp Tyr Leu Thr Arg His Tyr Leu Val Lys
   165 170
   Asn Lys Lys Leu Tyr Glu Phe Asn Asn Ser
   175 180
   Pro Tyr Glu Thr Gly Tyr Ile Lys Phe Ile
   185 190
   Glu Asn Glu Asn Ser Phe Trp Tyr Asp Met
   195 200
   Met Pro Ala Pro Gly Asp Lys Phe Asp Gln
   205 210
   Ser Lys Tyr Leu Met Met Tyr Asn Asp Asn
   215 220
   Lys Met Val Asp Ser Lys Asp Val Lys Ile
   225 230
   Glu Val Tyr Leu Thr Thr Lys Lys Lys
   235
(12) INFORMATION FOR SEQUENCE ID NO:11:
   (i) SEQUENCE CHARACTERISTICS:
      (A) LENGTH: 731
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:11:
   TAAGGAGAAT TAAAAATGAA TAAAAAATTA CTAATGAATT 40
   TTTTTATCGT AAGCCCTTTG TTGCTTGCGA CAACTGCTAC 80
   AGATTTTACC CCTGTTCCCT TATCATCTAA TCAAATAATC 120
   AAAACTGCAA AAGCATCTAC AAACGATAAT ATAAAGGATT 160
   TGCTAGACTG GTATAGTAGT GGGTCTGACA CTTTTACAAA 200
   TAGTGAAGTT TTAGATAATT CCAGAGGATC TATGCGTATA 240
   AAAAACACAG ATGGCAGCAT CAGCTTGATA ATTTTTCCGA 280
   GTCCTTATTA TAGCCCTGCT TTTACAAAAG GGGAAAAAGT 320
   TGACTTAAAC ACAAAAAGAA CTAAAAAAAG CCAACATACT 360
   AGCGAAGGAA CTTATATCCA TTTCCAAATA AGTGGCGTTA 400
   CAAATACTGA AAAATTACCT ACTCCAATAG AACTACCTTT 440
   AAAAGTTAAG GTTCATGGTA AAGATAGCCC CTTAAAGTAT 480
   GGGCCAAAGT TCGATAAAAA ACAATTAGCT ATATCAACTT 520
   TAGACTTTGA AATTCGTCAT CAGCTAACTC AAATACATGG 560
   ATTATATCGT TCAAGCGATA AAACGGGTGG TTATTGGAAA 600
   ATAACAATGA ATGACGGATC CACATATCAA AGTGATTTAT 640
   CTAAAAAGTT TGAATACAAT ACTGAAAAAC CACCTATAAA 680
   TATTGATGAA ATAAAAACTA TAGAAGCAGA AATTAATTAA 720
   TTTACCACTT T 731
(13) INFORMATION FOR SEQUENCE ID NO:12:
   (i) SEQUENCE CHARACTERISTICS:
      (A) LENGTH: 234
      (B) TYPE: Amino Acid
      (C) STRANDEDNESS: Unknown
      (D) TOPOLOGY: Unknown
   (ii) Molecule type: Peptide
   . (xi) SEQUENCE DESCRIPTION: SEQ ID NO:12:
(14) INFORMATION FOR SEQUENCE ID NO:13:
   (i) SEQUENCE CHARACTERISTICS:
      (A) LENGTH: 1095
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:13:
   ATCATTAAAT ATAATTAATT TTCTTTTAAT ATTTTTTTAA    40
   TTGAATATTT AAGATTATAA GATATATTTA AAGTGTATCT    80
   AGATACTTTT TGGGAATGTT GGATGAAGGA GATAAAAATG   120
   AATAAGAGTC GATTTATTTC ATGCGTAATT TTGATATTCG   160
   CACTTATACT AGTTCTTTTT ACACCCAACG TATTAGCAGA   200
   GAGCCAACCA GACCCTACGC CAGATGAGTT GCACAAAGCG   240
   AGTAAATTCA CTGGTTTGAT GGAAAATATG AAAGTTTTAT   280
   ATGATGATCA TTATGTATCA GCAACTAAAG TTAAGTCTGT   320
   AGATAAATTT AGGGCACATG ATTTAATTTA TAACATTAGT   360
   GATAAAAAAC TGAAAAATTA TGACAAAGTG AAAACAGAGT   400
   TATTAAATGA AGGTTTAGCA AAGAAGTACA AAGATGAAGT   440
   AGTTGATGTG TATGGATCAA ATTACTATGT AAACTGCTAT   480
   TTTTCATCCA AAGATAATGT AGGTAAAGTT ACAGGTGGCA   520
   AAACTTGTAT GTATGGAGGA ATAACAAAAC ATGAAGGAAA   560
   CCACTTTGAT AATGGGAACT TACAAAATGT ACTTATAAGA   600
   GTTTATGAAA ATAAAAGAAA CACAATTTCT TTTGAAGTGC   640
   AAACTGATAA GAAAAGTGTA ACAGCTCAAG AACTAGACAT   680
   AAAAGCTAGG AATTTTTTAA TTAATAAAAA AAATTTGTAT   720
   GAGTTTAACA GTTCACCATA TGAAACAGGA TATATAAAAT   760
   TTATTGAAAA TAACGGCAAT ACTTTTTGGT ATGATATGAT   800
   GCCTGCACCA GGCGATAAGT TTGACCAATC TAAATATTTA   840
   ATGATGTACA ACGACAATAA AACGGTTGAT TCTAAAAGTG     880
   TGAAGATAGA AGTCCACCTT ACAACAAAGA ATGGATAATG     920
   TTAATCCGAT TTTGATATAA AAAGTGAAAG TATTAGATAT     960
   ATTTGAAAGG TAAGTACTTC GGTGCTTGCC TTTTTAGGAT    1000
   GCATATATAT AGATTAAACC GCACTTCTAT ATTAATAGAA    1040
   AGTGCGGTTA TTTATACACT CAATCTAAAC TATAATAATT    1080
   GGAATCATCT TCAAA                               1095
(15) INFORMATION FOR SEQUENCE ID NO:14:
   (i) SEQUENCE CHARACTERISTICS:
      (A) LENGTH: 266
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:14:
   Met Asn Lys Ser Arg Phe Ile Ser Cys Val
                   5                   10
   Ile Leu Ile Phe Ala Leu Ile Leu Val Leu
                   15                  20
   Phe Thr Pro Asn Val Leu Ala Glu Ser Gln
                   25                  30
   Pro Asp Pro Thr Pro Asp Glu Leu His Lys
                   35                  40
   Ala Ser Lys Phe Thr Gly Leu Met Glu Asn
                   45                  50
   Met Lys Val Leu Tyr Asp Asp His Tyr Val
                   55                  60
   Ser Ala Thr Lys Val Lys Ser Val Asp Lys
                   65                  70
   Phe Arg Ala His Asp Leu Ile Tyr Asn Ile
                   75                  80
   Ser Asp Lys Lys Leu Lys Asn Tyr Asp Lys
   85 90
   Val Lys Thr Glu Leu Leu Asn Glu Gly Leu
   95 100
   Ala Lys Lys Tyr Lys Asp Glu Val Val Asp
   105 110
   Val Tyr Gly Ser Asn Tyr Tyr Val Asn Cys
   115 120
   Tyr Phe Ser Ser Lys Asp Asn Val Gly Lys
   125 130
   Val Thr Gly Gly Lys Thr Cys Met Tyr Gly
   135 140
   Gly Ile Thr Lys His Glu Gly Asn His Phe
   145 150
   Asp Asn Gly Asn Leu Gln Asn Val Leu Ile
   155 160
   Arg Val Tyr Glu Asn Lys Arg Asn Thr Ile
   165 170
   Ser Phe Glu Val Gln Thr Asp Lys Lys Ser
   175 180
   Val Thr Ala Gln Glu Leu Asp Ile Lys Ala
   185 190
   Arg Asn Phe Leu Ile Asn Lys Lys Asn Leu
   195 200
   Tyr Glu Phe Asn Ser Ser Phe Tyr Glu Thr
   205 210
   Gly Tyr Ile Lys Phe Ile Glu Asn Asn Gly
   215 220
   Asn Thr Phe Trp Tyr Asp Met Met Pro Ala
   225 230
   Pro Gly Asp Lys Phe Asp Gln Ser Lys Tyr
   235 240
   Leu Met Met Tyr Asn Asp Asn Lys Thr Val
   245 250
   Asp Ser Lys Ser Val Lys Ile Glu Val His
   255 260
   Leu Thr Thr Lys Asn Gly
   265
(16) INFORMATION FOR SEQUENCE ID NO:15:
   (i) SEQUENCE CHARACTERISTICS:
      (A) LENGTH: 1837
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:15:
   TCATGTTTGA CAGCTTATCA TCGATAAGCT TACTTTTCGA    40
   ATCAGGTCTA TCCTTGAAAC AGGTGCAACA TAGATTAGGG    80
   CATGGAGATT TACCAGACAA CTATGAACGT ATATACTCAC   120
   ATCACGCAAT CGGCAATTGA TGACATTGGA ACTAAATTCA   160
   ATCAATTTGT TACTAACAAG CAACTAGATT GACAACTAAT   200
   TCTCAACAAA CGTTAATTTA ACAACATTCA AGTAACTCCC   240
   ACCAGCTCCA TCAATGCTTA CCGTAAGTAA TCATAACTTA   280
   CTAAAACCTT GTTACATCAA GGTTTTTTCT TTTTGTCTTG   320
   TTCATGAGTT ACCATAACTT TCTATATTAT TGACAACTAA   360
   ATTGACAACT CTTCAATTAT TTTTCTGTCT ACTCAAAGTT   400
   TTCTTCATTT GATATAGTCT AATTCCACCA TCACTTCTTC   440
   CACTCTCTCT ACCGTCACAA CTTCATCATC TCTCACTTTT   480
   TCGTGTGGTA ACACATAATC AAATATCTTT CCGTTTTTAC   520
   GCACTATCGC TACTGTGTCA CCTAAAATAT ACCCCTTATC   560
   AATCGCTTCT TTAAACTCAT CTATATATAA CATATTTCAT   600
   CCTCCTACCT ATCTATTCGT AAAAAGATAA AAATAACTAT   640
   TGTTTTTTTT GTTATTTTAT AATAAAATTA TTAATATAAG   680
   TTAATGTTTT TTAAAAATAT ACAATTTTAT TCTATTTATA   720
   GTTAGCTATT TTTTCATTGT TAGTAATATT GGTGAATTGT   760
   AATAACCTTT TTAAATCTAG AGGAGAACCC AGATATAAAA    800
   TGGAGGAATA TTAATGGAAA ACAATAAAAA AGTATTGAAG    840
   AAAATGGTAT TTTTTGTTTT AGTGACATTT CTTGGACTAA    880
   CAATCTCGCA AGAGGTATTT GCTCAACAAG ACCCCGATCC    920
   AAGCCAACTT CACAGATCTA GTTTAGTTAA AAACCTTCAA    960
   AATATATATT TTCTTTATGA GGGTGACCCT GTTACTCACG   1000
   AGAATGTGAA ATCTGTTGAT CAACTTAGAT CTCACGATTT   1040
   AATATATAAT GTTTCAGGGC CAAATTATGA TAAATTAAAA   1080
   ACTGAACTTA AGAACCAAGA GATGGCAACT TTATTTAAGG   1120
   ATAAAAACGT TGATATTTAT GGTGTAGAAT ATTACCATCT   1160
   CTGTTATTTA TGTGAAAATG CAGAAAGGAG TGCATGTATC   1200
   TACGGAGGGG TAACAAATCA TGAAGGGAAT CATTTAGAAA   1240
   TTCCTAAAAA GATAGTCGTT AAAGTATCAA TCGATGGTAT   1280
   CCAAAGCCTA TCATTTGATA TTGAAACAAA TAAAAAAATG   1320
   GTAACTGCTC AAGAATTAGA CTATAAAGTT AGAAAATATC   1360
   TTACAGATAA TAAGCAACTA TATACTAATG GACCTTCTAA   1400
   ATATGAAACT GGATATATAA AGTTCATACC TAAGAATAAA   1440
   GAAAGTTTTT GGTTTGATTT TTTCCCTGAA CCAGAATTTA   1480
   CTCAATCTAA ATATCTTATG ATATATAAAG ATAATGAAAC   1520
   GCTTGACTCA AACACAAGCC AAATTGAAGT CTACCTAACA   1560
   ACCAAGTAAC TTTTTGCTTT TGGCAACCTT ACCTACTGCT   1600
   GGATTTAGAA ATTTTATTGC AATTCTTTTA TTAATGTAAA   1640
   AACCGCTCAT TTGATGAGCG GTTTTGTCTT ATCTAAAGGA   1680
   GCTTTACCTC CTAATGCTGC AAAATTTTAA ATGTTGGATT   1720
   TTTGTATTTG TCTATTGTAT TTGATGGGTA ATCCCATTTT   1760
   TCGACAGACA TCGTCGTGCC ACCTCTAACA CCAAAATCAT   1800
   AGACAGGAGC TTGTAGCTTA GCAACTATTT TATCGTC      1837
(17) INFORMATION FOR SEQUENCE ID NO:16:
   (i) SEQUENCE CHARACTERISTICS:
      (A) LENGTH: 251
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:16:

**SEQ ID NO:17**
   **sense primer for cloning SpeA**
   5' CTCG CAA GAG GTA CAT ATG CAA CAA GAC 3' (SEQ ID NO: ), sense primer to introduce a unique NdeI site;
**SEQ ID NO:18**
   **antisense primer for cloning SpeA**
   5' GCA GTA GGT AAG CTT GCC AAA AGC 3' (SEQ ID NO: )
SEQ ID NO:19
   1. **SpeA forward primer,** including NdeI site:
   5' GATATACATATGCAACAAGACCCCGATCCAAGCC 3' 34-mer
**SEQ ID NO:20**
   **SpeA reverse primer,** adds SpeB overlap
   5' GAGATTTAACAACTGGTTGCTTGGTTGTTAGGTAGAC 3' 37-mer
SEQ ID NO:21
   **3.SpeB forward primer**, adds SpeA overlap:
      5' GTCTACCTAACAACCAAGC A A C C A G T T G T T A A A T C T C 3' 37-mer SEQ ID NO:22
      **4. SpeB reverse primer**; adds stop site (Amber) and maintains BamHI site:
      5' GAATTCGGATCCGCTAGCCTACAACAG 3' 27-mer
SEQ ID NO:23
   SpeA(L42R)-SpeB(C47S) gene insert DNA sequence
SEQ ID NO:24
   Full-length SpeB polypeptide (Kagawa et al., PNAS 97:2235-2240. 2000): SEQ ID NO:25 SpeB clone used for fusion, mature polypeptide. Estimated Mᵣ= 28.75 kDa SEQ ID NO: 26 L42R SpeA mutant clone used for fusion. Estimated Mᵣ = 25.2 kDa SEQ ID NO:27 SpeA [L42R]-SpeB [C47S] fusion. Estimated Mᵣ= 54 kDa
SEQ ID NO:28
   **1. SpeA forward primer**, including NdeI site:
   5' GATATACATATGCAACAAGACCCCGATCCAAGCC 3' 34-mer
SEQ ID NO:29
   **2. SpeA- reverse primer**; kills NdeI site, adds SpeB overlap:
   5' CATGTGTATATCTCCTTCCTTGGTTGTTAGGTAGAC 3' 36-mer
SEQ ID NO:30
   **3.SpeB forward primer**; kills NdeI site, adds SpeA overlap:
   5' GTCTACCTAACAACCAAGGAAGGAGATATACACATG 3' 36-mer
SEQ ID NO:31
   **4. SpeB reverse primer**; adds stop site (Amber) and maintains BamHI site:
   5' GAATTCGGATCCGCTAGCCTACAACAG 3' 27-mer

## Claims

1. An isolated and purified superantigen toxin DNA fragment which has been altered such that binding of the encoded altered toxin to either the MHC class II or T cell antigen receptor is altered, wherein said superantigen is Streptococcal pyrogenic exotoxin A fused to Streptococcal pyrogenic exotoxin B, wherein said DNA fragment has the sequence of SEQ ID NO : 23.

2. An isolated and purified DNA fragment according to claim 1, wherein said fragment encodes the amino acid sequence of SEQ ID NO : 27.

3. A recombinant DNA construct comprising: (i) a vector, and (ii) an isolated and purified superantigen toxin DNA fragment which has been altered such that binding of the encoded altered toxin to either the MHC class II or T cell antigen receptor is altered, wherein said DNA fragment has the sequence according to SEQ ID NO : 23.

4. A recombinant DNA construct according to claim 3, wherein said DNA fragment encodes the amino acid sequence specified in SEQ ID NO : 27.

5. An isolated and purified superantigen toxin which has been altered such that binding of the encoded altered toxin to either the MHC class II or T cell antigen receptor is altered, wherein said toxin is Streptococcal pyrogenic exotoxin A in which position 42 has been altered to alanine or arginine, and said altered SpeA superantigen toxin is fused to Streptococcal pyrogenic exotoxin B in which position 47 has been altered to serine.

6. An altered superantigen according to claim 5 having amino acid SEQ ID NO : 27.

7. A multivalent vaccine against superantigen-associated bacterial infections comprising a combination of altered superantigen toxins selected from the group consisting essentially of TSST-1, SpeA, SEA, SEB, SEC-1, capable of eliciting protective antibodies against superantigen toxins in a pharmaceutically acceptable excipient in a pharmaceutically acceptable amount, the vaccine further comprising an altered SpeA superantigen toxin or peptide thereof fused to an altered SpeB superantigen toxin or peptide thereof, wherein position 42 of said SpeA superantigen toxin has been altered to alanine or arginine, and position 47 of said SpeB superantigen toxin has been altered to serine.

8. A vaccine comprising an isolated and purified SpeA superantigen toxin which has been altered such that binding of the encoded altered toxin to either the MHC class II or T cell antigen receptor is altered, wherein said altered SpeA superantigen toxin is fused to an altered SpeB superantigen toxin, wherein position 42 of said SpeA superantigen toxin has been altered to alanine or arginine, and position 47 of said SpeB superantigen toxin has been altered to serine, effective for the production of antigenic and immunogenic response resulting in the protection of a mammal against superantigen-associated bacterial infection, for use in the treatment or amelioration of a superantigen-associated bacterial infection.

9. An altered SpeA superantigen toxin from streptococcal bacteria fused to an altered SpeB superantigen toxin, wherein position 42 of said SpeA superantigen toxin has been altered to alanine or arginine, and position 47 of said SpeB superantigen toxin has been altered to serine, for use in the treatment or amelioration of a superantigen-associated bacterial infection by inhibiting adhesion of superantigen bacterial toxin to MAC class II or T cell receptors by competitive inhibition of these interactions.

10. Antisera isolated from individuals immunized with one or more altered SpeA superantigen toxins each fused to an altered SpeB superantigen toxin, wherein position 42 of said SpeA superantigen toxin has been altered to alanine or arginine, and position 47 of said SpeB superantigen toxin has been altered to serine.

11. Antisera according to claim 10 wherein position 42 of said SpeA superantigen toxin has been altered to alanine.

12. Antisera according to claim 10 wherein position 42 of said SpeA superantigen toxin has been altered to arginine.

13. An antibody which recognizes an altered SpeA superantigen toxin fused to an altered SpeB superantigen toxin, wherein position 42 of said SpeA superantigen toxin has been altered to alanine or arginine, and position 47 of said SpeB superantigen toxin has been altered to serine.

14. An antibody according to claim 13 wherein position 42 of said SpeA superantigen toxin has been altered to alanine.

15. An antibody according to claim 13 wherein position 42 of said SpeA superantigen toxin has been altered to arginine.

## Patentansprüche

1. Isoliertes und gereinigtes Superantigentoxin-DNA-Fragment, das so geändert worden ist, dass die Bindung des kodierten geänderten Toxins an entweder den MHC Klasse II- oder den T-Zellen-Antigen-Rezeptor geändert wird, wobei das Superantigen pyrogenes Streptokokken-Exotoxin A ist, das an pyrogenes Streptokokken-Exotoxin B fusioniert ist, wobei das DNA-Fragment die Sequenz von SEQ ID NO: 23 aufweist.

2. Isoliertes und gereinigtes DNA-Fragment nach Anspruch 1, wobei das Fragment für die Aminosäuresequenz von SEQ ID NO: 27 kodiert.

3. Rekombinantes DNA-Konstrukt umfassend: (i) einen Vektor und (ii) ein isoliertes und gereinigtes Superantigentoxin-DNA-Fragment, das so geändert worden ist, dass die Bindung des kodierten geänderten Toxins an entweder den MHC Klasse II- oder den T-Zellen-Antigen-Rezeptor geändert wird, wobei das DNA-Fragment die Sequenz gemäß SEQ ID NO: 23 aufweist.

4. Rekombinantes DNA-Konstrukt nach Anspruch 3, wobei das DNA-Fragment für die in SEQ ID NO: 27 spezifizierte Aminosäuresequenz kodiert.

5. Isoliertes und gereinigtes Superantigentoxin, das so geändert worden ist, dass die Bindung des kodierten geänderten Toxins an entweder den MHC Klasse II- oder den T-Zellen-Antigen-Rezeptor geändert wird, wobei das Toxin pyrogenes Streptokokken-Exotoxin A ist, in dem die Position 42 zu Alanin oder Arginin geändert worden ist und das geänderte SpeA-Superantigentoxin an pyrogenes Streptokokken-Exotoxin B fusioniert ist, in dem die Position 47 zu Serin geändert worden ist.

6. Geändertes Superantigen nach Anspruch 5, das die Aminosäure SEQ ID NO: 27 aufweist.

7. Mehrwertiger Impfstoff gegen mit Superantigen assoziierte Bakterieninfektionen, umfassend eine Kombination geänderter Superantigentoxine ausgewählt aus der Gruppe bestehend im Wesentlichen aus TSST-1, SpeA, SEA, SEB, SEC-1, das in der Lage ist, schützende Antikörper gegen Superantigentoxine hervorzurufen, in einem pharmazeutisch akzeptablen Trägerstoff in einer pharmazeutisch akzeptablen Menge, wobei der Impfstoff des Weiteren ein geändertes SpeA-Superantigentoxin oder ein Peptid davon umfasst, das an ein geändertes SpeB-Superantigentoxin oder Peptid davon fusioniert ist, wobei die Position 42 des SpeA-Superantigentoxins zu Alanin oder Arginin geändert worden ist und die Position 47 des SpeB-Superantigentoxins zu Serin geändert worden ist.

8. Impfstoff umfassend ein isoliertes und gereinigtes SpeA-Superantigentoxin, das so geändert worden ist, dass die Bindung des kodierten geänderten Toxins an entweder den MHC Klasse II- oder den T-Zellen-Antigen-Rezeptor geändert wird, wobei das geänderte SpeA-Superantigen an ein geändertes SpeB-Superantigentoxin fusioniert ist, wobei die Position 42 des SpeA-Superantigentoxins zu Alanin oder Arginin geändert worden ist und die Position 47 des SpeB-Superantigentoxins zu Serin geändert worden ist, der zur Erzeugung einer antigenen und immunogenen Reaktion wirksam ist, die zum Schutz eines Säugers gegen mit Superantigen assoziierter Bakterieninfektion führt, zur Verwendung bei der Behandlung oder Besserung einer mit Superantigen assoziierten Bakterieninfektion.

9. Geändertes SpeA-Superantigentoxin aus Streptokokkenbakterien, das an ein geändertes SpeB-Superantigentoxin fusioniert ist, wobei die Position 42 des SpeA-Superantigentoxins zu Alanin oder Arginin geändert worden ist und die Position 47 des SpeB-Superantigentoxins zu Serin geändert worden ist, zur Verwendung bei der Behandlung oder Besserung einer mit Superantigen assoziierter Bakterieninfektion durch Hemmen der Adhäsion von Superantigenbakterientoxin an MHC-Klasse II- oder T-Zellenrezeptoren durch kompetitive Hemmung dieser Wechselwirkungen.

10. Antiseren, die von Individuen isoliert worden sind, die mit einem oder mehreren geänderten SpeA-Superantigentoxinen immunisiert worden sind, die jedes an ein geändertes SpeB-Superantigentoxin fusioniert sind, wobei die Position 42 des SpeA-Superantigentoxins zu Alanin oder Arginin geändert worden ist und die Position 47 des SpeB-Superantigentoxins zu Serin geändert worden ist.

11. Antiseren nach Anspruch 10, wobei die Position 42 des SpeA-Superantigentoxins zu Alanin geändert worden ist.

12. Antiseren nach Anspruch 10, wobei die Position 42 des SpeA-Superantigentoxins zu Arginin geändert worden ist.

13. Antikörper, der ein geändertes SpeA-Superantigentoxinen erkennt, das an ein geändertes SpeB-Superantigentoxin fusioniert ist, wobei die Position 42 des SpeA-Superantigentoxins zu Alanin oder Arginin geändert worden ist und die Position 47 des SpeB-Superantigentoxins zu Serin geändert worden ist.

14. Antikörper nach Anspruch 13, wobei die Position 42 des SpeA-Superantigentoxins zu Alanin geändert worden ist.

15. Antikörper nach Anspruch 13, wobei die Position 42 des SpeA-Superantigentoxins zu Arginin geändert worden ist.

## Revendications

1. Fragment d'ADN de toxine superantigénique isolé et purifié, qui a été modifié de sorte que la liaison de la toxine modifiée codée au récepteurs soi du CMH de classe II soit d'un antigène de lymphocyte T est modifiée, dans lequel ledit superantigène est l'exotoxine pyrogène streptococcique A fusionnée à l'exotoxine pyrogène streptococcique B, ledit fragment d'ADN ayant la séquence de la SEQ ID NO: 23.

2. Fragment d'ADN isolé et purifié selon la revendication 1, ledit fragment codant la séquence d'acides aminés de la SEQ ID NO: 27.

3. Construction d'ADN recombinant comprenant: (i) un vecteur, et (ii) un fragment d'ADN de toxine superantigénique isolé et purifié qui a été modifié de sorte que la liaison de la toxine modifiée codée au récepteur soit du CMH de classe II soit d'un antigène de lymphocyte T est modifiée, dans laquelle ledit fragment d'ADN a la séquence selon la SEQ ID NO: 23.

4. Construction d'ADN recombinant selon la revendication 3, dans laquelle ledit fragment d'ADN code la séquence d'acides aminés spécifiée dans la SEQ ID NO: 27.

5. Toxine superantigénique isolée et purifiée qui a été modifiée de sorte que la liaison de la toxine modifiée codée au récepteur soit du CMH de classe II soit d'un antigène de lymphocyte T est modifiée, ladite toxine étant l'exotoxine pyrogène streptococcique A dont la position 42 a été modifiée en alanine ou arginine, et ladite toxine superantigénique SpeA modifiée est fusionnée à l'exotoxine pyrogène streptococcique B dont la position 47 a été modifiée en sérine.

6. Superantigène modifié selon la revendication 5, ayant la séquence d'acides aminés SEQ ID NO: 27.

7. Vaccin multivalent contre des infections bactériennes associées à un superantigène, comprenant une combinaison de toxines superantigéniques modifiées choisies dans le groupe essentiellement constitué par TSST-1, SpeA, SEA, SEB, SEC-1, capable de susciter des anticorps protecteurs contre les toxines superantigéniques, dans un excipient pharmaceutiquement acceptable en une quantité pharmaceutiquement acceptable, le vaccin comprenant en outre une toxine superantigénique SpeA modifiée ou un peptide de celle-ci fusionnée à une toxine superantigénique SpeB modifiée ou un peptide de celle-ci, dans lequel la position 42 de ladite toxine superantigénique SpeA a été modifiée en alanine ou arginine, et la position 47 de ladite toxine superantigénique SpeB a été modifiée en sérine.

8. Vaccin comprenant une toxine superantigénique SpeA isolée et purifiée qui a été modifiée de sorte que la liaison de la toxine modifiée codée au récepteur soit du CMH de classe II soit d'un antigène de lymphocyte T est modifiée, dans lequel ladite toxine superantigénique SpeA modifiée est fusionnée à une toxine superantigénique SpeB modifiée, dans lequel la position 42 de ladite toxine superantigénique SpeA a été modifiée en alanine ou arginine, et la position 47 de ladite toxine superantigénique SpeB a été modifiée en sérine, efficace pour la production d'une réponse antigénique et immunogène conduisant à la protection d'un mammifère contre une infection bactérienne associée à un superantigène, pour l'utilisation dans le traitement ou l'amélioration d'une infection bactérienne associée à un superantigène.

9. Toxine superantigénique SpeA modifiée de bactéries streptococciques fusionnée à une toxine superantigénique SpeB modifiée, dans laquelle la position 42 de ladite toxine superantigénique SpeA a été modifiée en alanine ou arginine, et la position 47 de ladite toxine superantigénique SpeB a été modifiée en sérine, pour l'utilisation dans le traitement ou l'amélioration d'une infection bactérienne associée à un superantigène en inhibant l'adhésion de la toxine bactérienne superantigénique à des récepteurs du CMH de classe II ou de lymphocytes T par inhibition compétitive de ces interactions.

10. Antisérums isolés à partir d'individus immunisés avec une ou plusieurs toxines superantigéniques SpeA modifiées, chacune étant fusionnée à une toxine superantigénique SpeB modifiée, dans lesquels la position 42 de ladite toxine superantigénique SpeA a été modifiée en alanine ou arginine, et la position 47 de ladite toxine superantigénique SpeB a été modifiée en sérine.

11. Antisérums selon la revendication 10, dans lesquels la position 42 de ladite toxine superantigénique SpeA a été modifiée en alanine.

12. Antisérums selon la revendication 10, dans lesquels la position 42 de ladite toxine superantigénique SpeA a été modifiée en arginine.

13. Anticorps qui reconnaît une toxine superantigénique SpeA modifiée fusionnée à une toxine superantigénique SpeB modifiée, dans lequel la position 42 de ladite toxine superantigénique SpeA a été modifiée en alanine ou arginine, et la position 47 de ladite toxine superantigénique SpeB a été modifiée en sérine.

14. Anticorps selon la revendication 13, dans lequel la position 42 de ladite toxine superantigénique SpeA a été modifiée en alanine.

15. Anticorps selon la revendication 13, dans lequel la position 42 de ladite toxine superantigénique SpeA a été modifiée en arginine.
